# EUROPEAN PATENT SPECIFICATION

(11) **EP 4 073 270 B1**
(45) Date of publication and mention of the grant of the patent: **21.01.2026**
(21) Application number: 20899255.2
(22) Date of filing: 08.12.2020
(51) Int. Cl.: C12Q 1/6858

(54) **RAPID AMPLIFICATION AND GENOTYPING OF NUCLEIC ACID SEQUENCES**
SCHNELLE AMPLIFIKATION UND GENOTYPISIERUNG VON NUKLEINSÄURESEQUENZEN
AMPLIFICATION ET GÉNOTYPAGE RAPIDES DE SÉQUENCES D'ACIDES NUCLÉIQUES

(30) Priority: 09.12.2019 SE 1951418
(43) Date of publication of application: 19.10.2022
(73) Proprietor: Laboratorios Maymó S.A.U., 08017 Barcelona (ES)
(72) Inventor: BELÁK, Sándor, 756 45 Uppsala (SE); GRANBERG, Fredrik, 756 52 Uppsala (SE)
(74) Representative: Barker Brettell Sweden AB
(86) International application number: PCT/SE2020/051175
(87) International publication number: WO 2021/118435

(56) References cited:
- EP-A1- 2 559 774
- WO-A1-2010/115154
- WO-A1-2011/056133
- WO-A1-2014/100866
- WO-A1-2018/108421
- WO-A1-2019/164885
- WO-A2-2005/042759
- WO-A2-2012/162267
- WO-A2-2012/162267
- US-A1- 2004 191 823
- US-A1- 2017 175 170
- US-B2- 10 202 640
- ALIA YACOUB ET AL: "Development of a novel real-time PCR-based strategy for simple and rapid molecular pathotyping of Newcastle disease virus", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 157, no. 5, 3 February 2012 (2012-02-03), pages 833 - 844, XP035048353, ISSN: 1432-8798, DOI: 10.1007/S00705-012-1231-0
- DUNBAR ET AL: "Applications of Luminex(R) xMAP(TM) technology for rapid, high-throughput multiplexed nucleic acid detection", CLINICA CHIMICA ACTA, ELSEVIER BV, AMSTERDAM, NL, vol. 363, no. 1-2, 1 January 2006 (2006-01-01), pages 71 - 82, XP027877582, ISSN: 0009-8981, [retrieved on 20060101]

## Description

### TECHNICAL FIELD

The present embodiments generally relate to amplification of nucleic acid sequences and genotyping of nucleic acid sequences.

### BACKGROUND

There is a general need for rapid and reliable methods for determination and classification of nucleotide sequences in the genome within genetic research and diagnosis of infectious diseases. For instance, different sequence variants of a locus in the genome of a microorganism may result in different phenotypes, such as a low vs. highly pathogenic microorganism.

A further example of determination and classification of nucleotide sequences is the need for rapid detection and determination of antibiotic resistance genes in various microorganisms. The development of antibiotic resistance and multi-resistant pathogens has greatly increased due to the excessive use of antibiotics in both human and veterinary medicine. It is now recognized as a very serious global health threat, which could result in a return to the pre-antibiotic era, as warned by the World Health Organization (WHO).

Traditional methods for antibiotic susceptibility testing (AST) of bacterial pathogens are cultivation based and require at least 24 hours of incubation after having the pathogens growing in pure culture. An accurate and more rapid multiplex determination of antibiotic resistance would greatly contribute to i) more effective treatment and infection control, ii) preventing loss of lives, and iii) reducing the economic losses caused by infectious bacterial diseases at a global scale. Since resistance genes are known to aggregate in plasmids, horizontal gene transfer can readily confer multi-resistance between even distantly related bacteria. An implication of this is that it might not be possible to determine the complete resistance profile of bacterial pathogens by using conventional molecular methods for rapid detection, such as real time polymerase chain reaction (PCR), because of their limited multiplexing capacity.

Document [1] discloses a rapid PCR-based molecular pathotyping of H5 and H7 avian influenza viruses (AIV). The method is a three-level semi-nested PCR assay designed to achieve highly multiplexed interrogation of the diverse cleavage site (CS) sequences in the hemagglutinin (HA) protein. Document [2] discloses a real-time PCR-based strategy for simple and rapid molecular pathotyping of Newcastle disease virus. The strategy involves a three-step semi-nested PCT comprising pre-amplification of the CS region of the F gene followed by a two-level duplex real-time PCR directly targeting the CS, combining detection and pathotyping in a single tube.

Document [3] discloses a method and a kit for genotyping N loci present in a sample in a target nucleic acid molecule. The method and kit address the problem of differentiating large sets of genomic sequences.

WO 2012/162267 discloses methods useful for incorporating one or more adaptors and/or nucleotide tag(s) and/or barcode nucleotide sequence(s) one, or typically more, target nucleotide sequences. Also described are methods and kits useful for amplifying one or more target nucleic acids in preparation for applications such as bidirectional nucleic acid sequencing.

### SUMMARY

It is a general objective to provide an efficient multiplex amplification and genotyping of nucleic acid sequences.

This and other objectives are met by embodiments as disclosed herein.

The present invention relates to methods and kits for amplification of a target nucleic acid sequence and for genotyping a locus in a target nucleic acid sequence as defined in the independent claims.

Further embodiments of the present invention are defined in the dependent claims.

The amplification of a target nucleic acid sequence is a primer-dependent amplification involving two amplification steps. The first amplification step is a selective amplification of the target nucleic acid sequence using a set of selection primers and a first set of amplification primers in a primer-dependent enzymatic reaction to yield an amplicon. The second amplification step is an amplification and barcoding of the amplicon using a barcoding primer and a second set of amplification primers in a primer-dependent enzymatic reaction to yield a barcoded amplicon.

The set of selection primers comprises a forward selection primer comprising, from a 5' end to a 3' end, a first non-genotype specific amplification sequence and a first recognition sequence complementary to a first segment of the target nucleic acid sequence and a reverse selection primer comprising, from a 5' end to a 3' end, a second non-genotype specific amplification sequence and a second recognition sequence complementary to a second segment of the target nucleic acid sequence.

The first set of amplification primers comprises a forward amplification primer corresponding or complementary to the first non-genotype specific amplification sequence and a reverse amplification primer corresponding or complementary to the second non-genotype specific amplification sequence.

The barcoding primer comprises, from a 5' end to a 3' end i) the first non-genotype specific amplification sequence or a third non-genotype specific amplification sequence, a barcode sequence and the first recognition sequence or a third recognition sequence complementary to a first segment of the amplicon, or ii) the second non-genotype specific amplification sequence or a third non-genotype specific amplification sequence, a barcode sequence and the second recognition sequence or a fourth recognition sequence complementary to a second segment of the amplicon, or iii) the barcoding primer comprises a forward barcoding primer and a reverse barcoding primer. The forward barcoding primer then comprises, from a 5' end to a 3' end, the first non-genotype specific amplification sequence or a third non-genotype specific amplification sequence, a first barcode sequence and the first recognition sequence or a third recognition sequence complementary to a first segment of the amplicon and the reverse barcoding primer comprises, from a 5' end to a 3' end, the second non-genotype specific amplification sequence or a third non-genotype specific amplification sequence, a second barcode sequence and the second recognition sequence or a fourth recognition sequence complementary to a second segment of the amplicon.

The second set of amplification primers comprises i) a forward amplification primer corresponding or complementary to the first non-genotype specific amplification sequence or the third non-genotype specific amplification sequence, and a reverse amplification primer corresponding or complementary to the second non-genotype specific amplification sequence, or ii) a forward amplification primer corresponding or complementary to the first non-genotype specific amplification sequence, and a reverse amplification primer corresponding or complementary to the second non-genotype specific amplification sequence or the third non-genotype specific amplification sequence, or iii) a forward amplification primer corresponding or complementary to the first non-genotype specific amplification sequence or the third non-genotype specific amplification sequence and a reverse amplification primer corresponding or complementary to the second non-genotype specific amplification sequence or the third non-genotype specific amplification sequence.

The first set of amplification primers is present in a concentration of at least 100:1 with regard to a concentration of the set of selection primers and the second set of amplification primers is present in a concentration of at least 100:1 with regard to a concentration of the barcoding primer.

The comparatively much lower concentration of the set of selection primers and the barcoding primer as compared to the first and second sets of amplification primers enables a high degree of multiplexing, i.e., running amplification or genotyping of different target nucleic acid sequences in a same reaction vessel.

### BRIEF DESCRIPTION OF THE DRAWINGS

The embodiments, together with further objects and advantages thereof, may best be understood by making reference to the following description taken together with the accompanying drawings, in which:
Fig. 1 is a flow chart illustrating a method of amplification of a target nucleic acid sequence according to an embodiment;
Fig. 2 schematically illustrates a target nucleic acid sequence together with a set of selection primers and a first set of amplification primers according to an embodiment;
Figs. 3A to 3I illustrate amplicons together with a barcoding primer and a second set of amplification primers according to various embodiments;
Figs. 4A to 4I illustrate barcoded amplicons according to various embodiments obtained according to the embodiments shown in Figs. 3A to 3I;
Fig. 5 schematically illustrates optional pre-amplification, selective amplification and barcoding and amplification in a method of amplification of a target nucleic acid sequence according to an embodiment;
Fig. 6 is a flow chart illustrating a method of genotyping a locus in a target nucleic acid sequence according to an embodiment;
Fig. 7 is a flow chart illustrating an additional, optional step of the method shown in Fig. 6 according to an embodiment;
Figs 8A and 8B schematically illustrate optional pre-amplification, selective amplification, barcoding and amplification and genotyping in a method of genotyping a locus in a target nucleic acid sequence according to an embodiment;
Figs. 9A and 9B schematically illustrate optional pre-amplification, selective amplification, barcoding and amplification and genotyping in a method of genotyping a locus in a target nucleic acid sequence according to another embodiment;
Fig. 10 schematically illustrates optional pre-amplification and selective amplification in a method of genotyping a locus in a target nucleic acid sequence according to a further embodiment;
Figs. 11A to 11C schematically illustrate an embodiment of preparing labelled detection probes suitable for detection and reading with LUMINEX^{®};
Fig. 12 illustrates LUMINEX^{®} results of an original plasmid used for optimization; and
Figs. 13A-13F illustrate LUMINEX^{®} results from six bacterial samples.

### DETAILED DESCRIPTION

Throughout the drawings, the same reference numbers are used for similar or corresponding elements.

The present embodiments generally relate to amplification of nucleic acid sequences and genotyping of nucleic acid sequences.

The amplification of target nucleic acid sequences, also denoted target nucleotide sequences herein, is a primer-dependent amplification involving two amplification steps. In the first step, a selective amplification of the target nucleic acid sequence is performed in order to increase the sensitivity of the amplification method, i.e., enable amplification of target nucleic acid sequences even in low concentrations or numbers. A second step comprises amplification and barcoding. This second step achieves a selective amplification and tag labelling in order to increase the specificity of the amplification method, i.e., enable amplification of target nucleic acid sequences but no or low amplifications of other nucleic acid sequences.

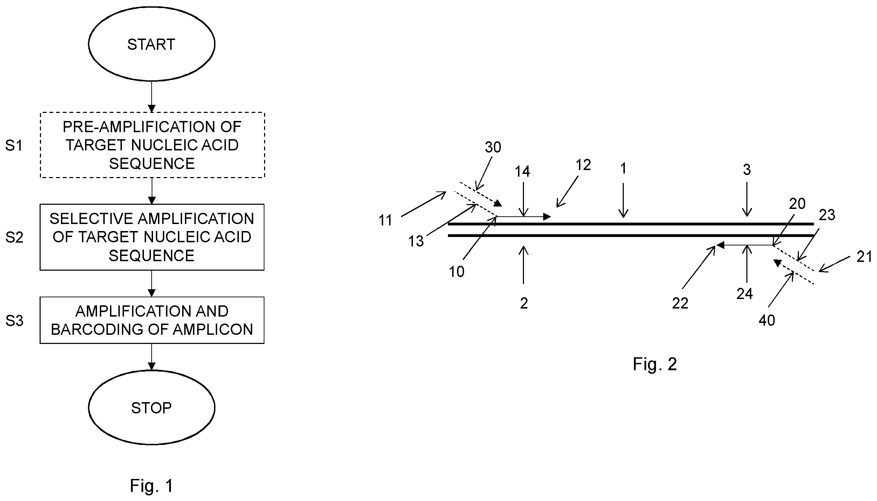

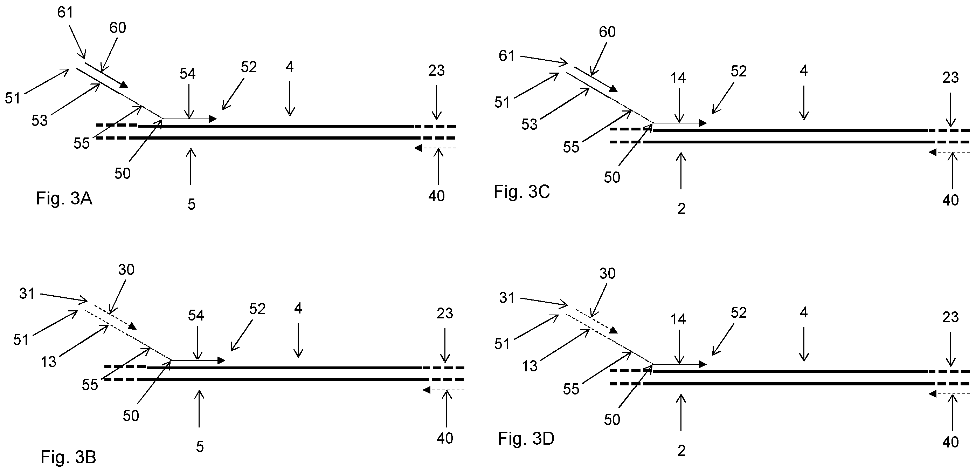

Fig. 1 is a flow chart illustrating a method of amplification of a target nucleic acid sequence 1 according to an embodiment, see also Figs. 2, 3A-3I. The method comprises selective amplification, in step S2, of the target nucleic acid sequence 1 using a set of selection primers 10, 20 and a first set of amplification primers 30, 40 in a primer-dependent enzymatic reaction to yield an amplicon 4.

According to the embodiments, the set of selection primers 10, 20 comprises a forward selection primer 10 and a reverse selection primer 20. The forward selection primer 10 comprises, from a 5' end 11 to a 3' end 12, a first non-genotype specific amplification sequence 13 and a first recognition sequence 14 complementary to a first segment 2 of the target nucleic acid sequence 1. The reverse selection primer 20 comprises, from a 5' end 21 to a 3' end 22, a second non-genotype specific amplification sequence 23 and a second recognition sequence 24 complementary a second segment 3 of the target nucleic acid sequence 1.

The first set of amplification primers 30, 40 comprises a forward amplification primer 30 and a reverse amplification primer 40. The forward amplification primer 30 is corresponding or complementary, preferably corresponding, to the first non-genotype specific amplification sequence 13 and the reverse amplification primer 40 is corresponding or complementary, preferably corresponding, to the second non-genotype specific amplification sequence 23.

The first set of amplification primers 30, 40 is present in a concentration of at least 100:1 with regard to a concentration of the set of selection primers 10, 20.

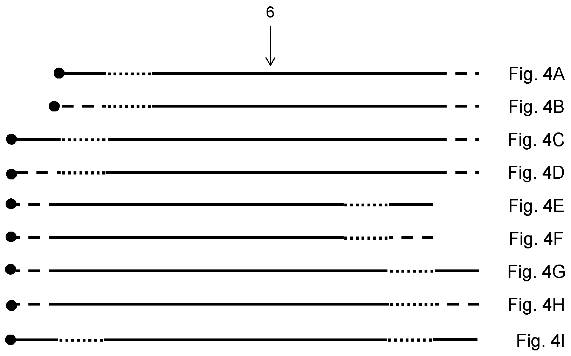

The method also comprises amplification and barcoding, in step S3, of the amplicon 4 using a barcoding primer 50 and a second set of amplification primers 30, 60; 40, 70 in a primer-dependent enzymatic reaction to yield a barcoded amplicon 6, see Figs. 4A-4I.

The barcoding primer 50 comprises, in an embodiment and from a 5' end 51 to a 3' end 52, the first non-genotype specific amplification sequence 13 or a third non-genotype specific amplification sequence 53, a barcode sequence 55 and the first recognition sequence 14 or a third recognition sequence 54 complementary to a first segment 5 of the amplicon 4 (and thereby of a corresponding segment in the target nucleic acid sequence 1). In this embodiment, the barcoding primer 50 is a so-called forward barcoding primer 50 as shown in Figs. 3A-3D.

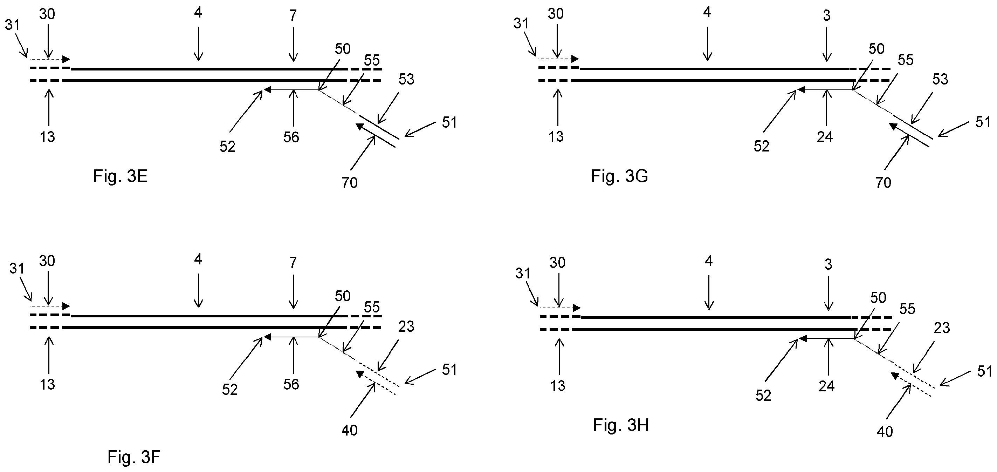

In another embodiment, the barcoding primer 50 comprises, from the 5' end 51 to the 3' end 52, the second non-genotype specific amplification sequence 23 or a third non-genotype specific amplification sequence 53, a barcode sequence 55 and the second recognition sequence 24 or a fourth recognition sequence 56 complementary to a second segment 7 of the amplicon 4 (and thereby of a corresponding segment in the target nucleic acid sequence 1). In this another embodiment, the barcoding primer 50 is a so-called reverse barcoding primer 50 as shown in Figs. 3E-3H.

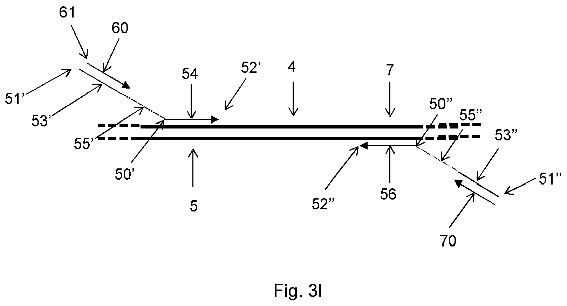

In a further embodiment, a first barcoding primer 50' and a second barcoding primer 50" are used, see Fig. 3I. In such an embodiment, the first barcoding primer 50' comprises, from the 5' end 51' to the 3' end 52', the first non-genotype specific amplification sequence 13 or a third non-genotype specific amplification sequence 53', a first barcode sequence 55' and the first recognition sequence 14 or a third recognition sequence 54 complementary to a first segment 5 of the amplicon 4 (and thereby of a corresponding segment in the target nucleic acid sequence 1). The second barcoding primer 50" comprises, from the 5' end 51" to the 3' end 52", the second non-genotype specific amplification sequence 23 or a third non-genotype specific amplification sequence 53", a second barcode sequence 55" and the second recognition sequence 24 or a fourth recognition sequence 56 complementary to a second segment 7 of the amplicon 4 (and thereby of a corresponding segment in the target nucleic acid sequence 1). In this embodiment, the first barcoding primer 50' is a so-called forward barcoding primer whereas the second barcoding primer 50" is a so-called reverse barcoding primer.

The second set of amplification primers 30, 60; 40, 70 comprises a forward amplification primer 30, 60 and a reverse amplification primer 40, 70. In the embodiment with forward barcoding primer 50, see Figs. 3A-3D, the forward amplification primer 30, 60 is corresponding or complementary, preferably corresponding, to the first non-genotype specific amplification sequence 13 or the third non-genotype specific amplification sequence 53 and the reverse amplification primer 40 is corresponding or complementary, preferably corresponding, to the second non-genotype specific amplification sequence 23. In the another embodiment with reverse barcoding primer 50, see Figs. 3E-3H, the forward amplification primer 30 is corresponding or complementary, preferably corresponding, to the first non-genotype specific amplification sequence 13 and the reverse amplification primer 40, 70 is corresponding or complementary, preferably corresponding, to the second non-genotype specific amplification sequence 23 or the third non-genotype specific amplification sequence 53. In the further embodiment with forward and reverse barcoding primers 50', 50", see Fig. 3I, the forward amplification primer 30, 60 is corresponding or complementary, preferably corresponding, to the first non-genotype specific amplification sequence 13 or the third non-genotype specific amplification sequence 53' and the reverse amplification primer 40, 70 is corresponding or complementary, preferably corresponding, to the second non-genotype specific amplification sequence 23 of the third non-genotype specific amplification sequence 53".

The second set of amplification primers 30, 60; 40, 70 is present in a concentration of at least 100:1 with regard to a concentration of the barcoding primer 50, 50', 50".

Thus, the amplification method as shown in Fig. 1 comprises two primer-dependent enzymatic reactions. In the first primer-dependent enzymatic reaction, step S2, a selective amplification of the target nucleic acid sequence 1 takes place to yield the amplicon 4. The second primer-dependent enzymatic reaction, step S3, comprises an amplification and barcoding of the amplicon 4 from step S2 to yield the barcoded amplicon 6.

Both primer-dependent enzymatic reactions involve using a respective set of amplification primers 30, 40; 30, 60, 40, 70 that is present in a comparatively much higher concentration or amount, i.e., at least 100:1, with regard to the concentration or amount of the set of selection primers 10, 20 in step S2 and the concentration or amount of the barcoding primer 50 or primers 50', 50" in step S3.

Thus, the selective amplification of the target nucleic acid sequence 1 in step S2 is performed using selection primers 10, 20 in low concentration and composed of a respective recognition sequences 14, 24 and forward and reverse non-genotype specific sequences 13, 23 for amplification as well as corresponding or complementary amplification primers 30, 40 in high concentration.

The comparatively much lower concentration of the selection primers 10, 20 as compared to the amplification primers 30, 40 enables a high degree of multiplexing, which is further described herein. This is possible since the comparatively low concentration of selection primers 10, 20 reduces the risk of interactions between selection primers 10, 20. Accordingly, a plurality of different selection primers 10, 20 can be used simultaneously in the amplification method.

The recognition sequences 14, 24 are target specific in terms of being complementary to and thereby capable of hybridizing to respective segments 2, 3 of the target nucleic acid sequence 1. In a particular embodiment, first recognition sequence 14 of the forward selection primer 10 is complementary to the first segment 2 of the target nucleic acid sequence 1 in the sense strand, whereas the second recognition sequence 24 of the reverse selection primer 20 is complementary to the second segment 3 of the target nucleic sequence 1 in the antisense strand. In another embodiment, first recognition sequence 14 is complementary to the first segment 2 of the target nucleic acid sequence 1 in the antisense strand and the second recognition sequence 24 is complementary to the second segment 3 of the target nucleic sequence 1 in the sense strand.

In an embodiment, the forward amplification primer 30 is corresponding to the first non-genotype specific amplification sequence 13 and the reverse amplification primer 40 is corresponding to the second non-genotype specific amplification sequence 23. In another embodiment, the forward amplification primer 30 is complementary to the first non-genotype specific amplification sequence 13 and the reverse amplification primer 40 is complementary to the second non-genotype specific amplification sequence 23.

In an embodiment, the forward amplification primer 30, 60 is corresponding to the first non-genotype specific amplification sequence 13 or the third non-genotype specific amplification sequence 53, 53' and the reverse amplification primer 40, 70 is corresponding to the second non-genotype specific amplification sequence 23 or the third non-genotype specific amplification sequence 53, 53". In another embodiment, the forward amplification primer 30, 60 is complementary to the first non-genotype specific amplification sequence 13 or the third non-genotype specific amplification sequence 53, 53' and the reverse amplification primer 40, 70 is complementary to the second non-genotype specific amplification sequence 23 or the third non-genotype specific amplification sequence 53, 53".

Hybridization or hybridization condition denotes the process in which single-stranded nucleic acid sequences anneal to complementary nucleic acid sequences. Such annealing between complementary nucleic acid sequences is dependent on several parameters including, for instance, ionic strength, temperature, length of the selection primers 10, 20, or rather the recognition sequences 14, 24 thereof, and G-C-nucleotides content of the recognition sequences 14, 24.

Complementary as user herein refers both to complete complementarity of nucleic acid sequences, in some cases referred to as an identical sequence, as well as complementarity sufficient to achieve the desired binding of nucleic acid sequences. Complementary refers to the standard base pairing rules between G-C, A-T and A-U. Certain nucleotides not commonly found in natural nucleic acid sequences or chemically synthesized may be included in the nucleic sequences described herein. Complementarity need not be perfect. In clear contrast, stable duplexes may contain mismatched base pairs, degenerative, or unmatched nucleotides.

Furthermore, an amplification primer 30, 40 corresponding to a nucleic acid sequence 13, 23, 53 as used herein refers both to complete identity of nucleic acid sequences, i.e., the nucleic acid sequence of the amplification primer 30, 40 being identical or equal to the nucleic acid sequence 13, 23, 53, as well as corresponding sufficiently so as that a nucleic acid molecule that is complementary to and capable of hybridizing to the nucleic acid sequence 13, 23, 53 is also complementary to and capable of hybridizing to the amplification primer 30, 40. Hence, corresponding to as used herein also encompasses the case where there may be one or few mismatched between the nucleotides of the amplification primer 30, 40 and its corresponding nucleic acid sequence 13, 23, 53.

The non-genotype specific amplification sequences 13, 23 are not target specific, i.e., are not complementary to and do not efficiently hybridize to the target nucleic acid sequence 1. These non-genotype specific amplification sequences 13, 23 are also referred to herein as first common amplification sequence 13 and second common amplification sequence 23 to indicate that they are not specific to the particular target nucleic acid sequence 1.

The amplification and barcoding of the amplicon 4 in step S3 are performed using the barcoding primer 50 in low concentration as well as corresponding or complementary amplification primers 30, 60; 40, 70 in high concentration.

The comparatively much lower concentration of the barcoding primer 50 as compared to the amplification primers 30, 60; 40, 70 enables a high degree of multiplexing, which is further described herein. This is possible since the comparatively low concentration of barcoding primer 50 reduces the risk of interactions between barcoding primers 50. Accordingly, a plurality of different barcoding primers 50 can be used simultaneously in the amplification method.

Figs. 3A-3I illustrate various embodiments of barcoding primers 50 that can be used in the amplification and barcoding step S3 in Fig. 1. Figs. 4A-4I illustrate the corresponding barcoded amplicons 5 obtained according to the embodiments shown in Figs. 3A-3I.

In a first embodiment, see Figs. 3A, the barcoding primer 50 comprises, from the 5' end 51 to the 3' end 52, the third non-genotype specific amplification sequence 53, the barcode sequence 55 and the third recognition sequence 54 complementary to the first segment 5 of the amplicon 4 (and thereby of a corresponding segment in the target nucleic acid sequence 1).

In this first embodiment, the forward amplification primer 60 of the second set of amplification primers 60, 40 is corresponding or complementary to the third non-genotype specific amplification sequence 53 and the reverse amplification primer 40 is corresponding or complementary to the second non-genotype specific amplification sequence 23.

Fig. 4A illustrates the barcoded amplicon 6 obtained according to this first embodiment.

In a second embodiment, see Figs. 3B, the barcoding primer 50 comprises, from the 5' end 51 to the 3' end 52, the first non-genotype specific amplification sequence 13, the barcode sequence 55 and the third recognition sequence 54 complementary to the first segment 5 of the amplicon 4 (and thereby of a corresponding segment in the target nucleic acid sequence 1).

In this second embodiment, the forward amplification primer 30 of the second set of amplification primers 30, 40 is corresponding or complementary to the first non-genotype specific amplification sequence 13 and the reverse amplification primer 40 is corresponding or complementary to the second non-genotype specific amplification sequence 23.

Fig. 4B illustrates the barcoded amplicon 6 obtained according to this second embodiment.

In a third embodiment, see Figs. 3C, the barcoding primer 50 comprises, from the 5' end 51 to the 3' end 52, the third non-genotype specific amplification sequence 53, the barcode sequence 55 and the first recognition sequence 14 complementary to the first segment 2 of the target nucleic acid sequence 1 (and thereby of a corresponding segment in the amplicon 4).

In this third embodiment, the forward amplification primer 60 of the second set of amplification primers 60, 40 is corresponding or complementary to the third non-genotype specific amplification sequence 53 and the reverse amplification primer 40 is corresponding or complementary to the second non-genotype specific amplification sequence 23.

Fig. 4C illustrates the barcoded amplicon 6 obtained according to this third embodiment.

In a fourth embodiment, see Figs. 3D, the barcoding primer 50 comprises, from the 5' end 51 to the 3' end 52, the first non-genotype specific amplification sequence 13, the barcode sequence 55 and the first recognition sequence 14 complementary to the first segment 2 of the target nucleic acid sequence 1 (and thereby of a corresponding segment in the amplicon 4).

In this fourth embodiment, the forward amplification primer 30 of the second set of amplification primers 30, 40 is corresponding or complementary to the first non-genotype specific amplification sequence 13 and the reverse amplification primer 40 is corresponding or complementary to the second non-genotype specific amplification sequence 23.

Fig. 4D illustrates the barcoded amplicon 6 obtained according to this fourth embodiment.

In the first to fourth embodiments above and shown in Figs. 3A-3D, 4A-4D the barcoding primer 50 is a forward barcoding primer. In the following fifth to eighth embodiments shown in Figs. 3E-3H, 4E-4H the barcoding primer 50 is a reverse barcoding primer.

In a fifth embodiment, see Figs. 3E, the barcoding primer 50 comprises, from the 5' end 51 to the 3' end 52, the third non-genotype specific amplification sequence 53, the barcode sequence 55 and the fourth recognition sequence 56 complementary to the second segment 7 of the amplicon 4 (and thereby of a corresponding segment in the target nucleic acid sequence 1).

In this fifth embodiment, the forward amplification primer 30 of the second set of amplification primers 30, 70 is corresponding or complementary to the first non-genotype specific amplification sequence 13 and the reverse amplification primer 70 is corresponding or complementary to the third non-genotype specific amplification sequence 53.

Fig. 4E illustrates the barcoded amplicon 6 obtained according to this fifth embodiment.

In a sixth embodiment, see Figs. 3F, the barcoding primer 50 comprises, from the 5' end 51 to the 3' end 52, the second non-genotype specific amplification sequence 23, the barcode sequence 55 and the fourth recognition sequence 56 complementary to the second segment 7 of the amplicon 4 (and thereby of a corresponding segment in the target nucleic acid sequence 1).

In this sixth embodiment, the forward amplification primer 30 of the second set of amplification primers 30, 40 is corresponding or complementary to the first non-genotype specific amplification sequence 13 and the reverse amplification primer 40 is corresponding or complementary to the second non-genotype specific amplification sequence 23.

Fig. 4F illustrates the barcoded amplicon 6 obtained according to this sixth embodiment.

In a seventh embodiment, see Figs. 3G, the barcoding primer 50 comprises, from the 5' end 51 to the 3' end 52, the third non-genotype specific amplification sequence 53, the barcode sequence 55 and the second recognition sequence 24 complementary to the second segment 3 of the target nucleic acid sequence 1 (and thereby of a corresponding segment in the amplicon 4).

In this seventh embodiment, the forward amplification primer 30 of the second set of amplification primers 30, 70 is corresponding or complementary to the first non-genotype specific amplification sequence 13 and the reverse amplification primer 70 is corresponding or complementary to the third non-genotype specific amplification sequence 53.

Fig. 4G illustrates the barcoded amplicon 6 obtained according to this seventh embodiment.

In an eighth embodiment, see Figs. 3H, the barcoding primer 50 comprises, from the 5' end 51 to the 3' end 52, the second non-genotype specific amplification sequence 23, the barcode sequence 55 and the second recognition sequence 24 complementary to the second segment 3 of the target nucleic acid sequence 1 (and thereby of a corresponding segment in the amplicon 4).

In this eighth embodiment, the forward amplification primer 30 of the second set of amplification primers 30, 40 is corresponding or complementary to the first non-genotype specific amplification sequence 13 and the reverse amplification primer 40 is corresponding or complementary to the second non-genotype specific amplification sequence 23.

Fig. 4H illustrates the barcoded amplicon 6 obtained according to this eighth embodiment.

The third non-genotype specific amplification sequence 53 in a reverse barcoding primer 50 could be the same or different as compared to the corresponding third non-genotype specific amplification sequence 53 in a forward barcoding primer 50.

As previously discussed herein, it is possible to combine a forward barcoding primer 50' with a reverse barcoding primer 50", an illustrative example of which is shown in Fig. 3I with the resulting barcoded amplicon 6 shown in Fig. 4I. Generally, the forward barcoding primer 50' could be any forward barcoding primer 50 as shown in Figs. 3A to 3D and the reverse barcoding primer 50" could be any reverse barcoding primer 50 as shown in Figs. 3E to 3H.

As is seen in Figs. 4A to 4I showing the barcode sequence as a dotted line in the barcoded amplicon 6, the barcode sequence is closer to the 5' end of the barcoded amplicon 6, having a solid dot in the figures, when using a forward barcoding primer, i.e., Figs. 4A-4D, 4I, as compared to using a reverse barcoding primer, i.e., Figs. 4E-4H. In some embodiments, it is generally preferred to have the barcode sequence close to the 5' end, in particular for long amplicons, which is further described herein.

In an embodiment, the method comprises an optional pre-amplification step S1 as shown in Fig. 1. In such a case, step S1 comprises pre-amplification of the target nucleic acid sequence 1 in a primer-dependent enzymatic reaction.

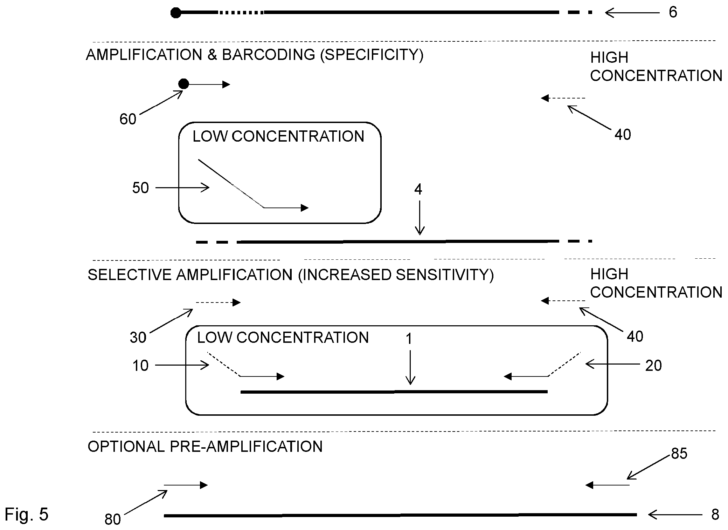

The optional pre-amplification preferably involves using a third set of amplification primers 80, 85 comprising a forward amplification primer 80 and a reverse amplification primer 85 as shown in the lower part of Fig. 5. The third set of amplification primers 80, 85 thereby amplifies the target nucleic acid sequence 1 from an original or starting nucleic acid molecule 8 to increase the copy number of the target nucleic acid sequence 1 prior to the selective amplification and the amplification and barcoding.

Fig. 5 also schematically illustrates the relative concentrations or amounts of the primers 10, 20, 30, 40, 50, 60, 80, 85 employed in the amplification method. Thus, amplification primers 30, 40, 60, 80, 85 are present in a comparatively high concentration as compared to the selection primers 10, 20 and the barcoding primer 50.

The amplification method of the embodiments enables multiplexing, i.e. using a plurality of different selection primers 10, 20 and barcoding primers 50 in a same reaction container or vessel. This is possible due to the large difference, i.e., at least 100:1, between the concentrations of the amplification primers 30, 40, 60, 70 and the selection primers 10, 20 and the barcoding primer 50 used in the primer-dependent enzymatic reactions of steps S2 and S3. Such a multiplexing is in particular useful in connection with genotyping a locus in a target nucleic acid sequence. Hence, the amplification method of the embodiments can advantageously be used in such a locus genotyping.

Genotyping a locus as used herein comprises determining the nucleotide sequence of a locus, or at least a portion thereof, in a target nucleic acid sequence, such as a gene; a gene control element, such as a promoter, etc.; or a portion thereof. The genotyping can advantageously be used to determine the nucleotide sequence of a polymorphic locus, site or other region of a nucleic acid molecule. Polymorphism defines the presence of multiple, i.e., at least two, alleles of a nucleic acid sequence, such as a gene, a gene control element, or a portion thereof. Such a polymorphic locus can be in the form of Single Nucleotide Polymorphism (SNP), i.e., a variation in a single nucleotide that occurs at a specific position in the genome; or can involve variations in multiple nucleotides, such as in the form of nucleotide insertions, nucleotide deletions and/or nucleotide substitutions.

The locus of the target nucleic acid sequence may define a particular property or characteristic, such as different phenotypes, e.g., a low vs. high pathogenic microorganism, resistance to antibiotics, etc.

The target nucleotide sequence of the amplification method and the genotyping method could be any nucleotide or nucleic acid sequence or molecule comprising or consisting of a Sequence Of Interest (SOI) or a sequence to be amplified and optionally further processed, such as genotyped. The target nucleotide sequence may, thus, be a single-stranded or double-stranded DNA sequence, a RNA sequence, a genomic DNA sequence, a cDNA sequence, or indeed any other nucleotide or nucleic acid sequence from any source. The target nucleotide sequence is preferably present in a sample, such as biological sample, from a subject, such as an animal subject, preferably a mammal subject, and more preferably a human subject. The invention can be used for both medical and veterinary purposes. In the latter case, the sample is preferably from a mammal subject, such as pig, cattle, horse, cat, dog, rat, mouse, goat, sheep, guinea pig, rabbit, as illustrative, but non-limiting examples. The invention can also be used with biological samples from birds, such as turkey, grouse, or chicken or other poultry. In such a case, the biological sample could be body fluid sample, such as a blood sample, a blood plasma sample, a saliva sample, a cerebrospinal fluid sample, or an endometrial fluid sample, or a body tissue sample, such as a biopsy sample. Also feces samples could be used as biological sample.

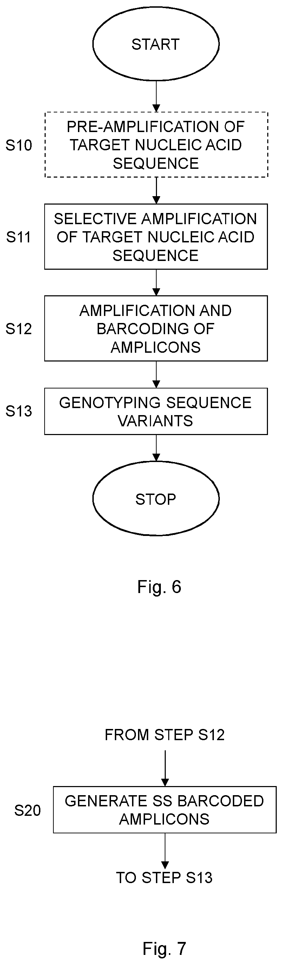

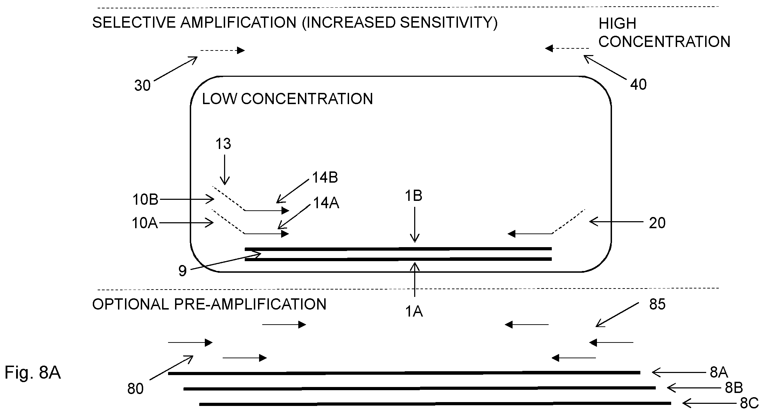

Fig. 6 is a flow chart illustrating a method of genotyping a locus 9; 9A, 9B, such as region or marker region, in a target nucleic acid sequence 1A, 1B, see Figs. 8A-10. The locus 9; 9A, 9B has N sequence variants, such as alleles, corresponding to N genotypes, wherein *N*≥2. The method comprises selective amplification of the target nucleic acid sequence 1A, 1B in step S11 using N sets of selection primers 10A, 10B, 20; 10, 20A, 20B; 10A, 10B, 20A, 20B and a first set of amplification primers 30, 40 in a primer-dependent enzymatic reaction to yield amplicons 4A, 4B.

Each set of selection primers 10A, 10B, 20; 10, 20A, 20B; 10A, 10B, 20A, 20B of the N sets of selection primers 10A, 10B, 20; 10, 20A, 20B; 10A, 10B, 20A, 20B comprises a respective forward selection primer 10A, 10B and a common reverse selection primer 20, a common forward selection primer 10 and a respective reverse selection primer 20A, 20B or a respective forward selection primer 10A, 10B and a respective reverse selection primer 20A, 20B.

The respective forward selection primer 10A, 10B comprises, from a 5' end 11 to a 3' end 12, a first non-genotype specific amplification sequence 13 and a respective first locus recognition sequence 14A, 14B complementary to a respective sequence variant of the locus 9; 9A. The common reverse selection primer 20 comprises, from a 5' end 21 to a 3' end 22, a second non-genotype specific amplification sequence 23 and a recognition sequence 24 complementary to a second segment 3 of the target nucleic acid sequence 1. The common forward selection primer 10 comprises, from a 5' end 11 to a 3' end 12, a first non-genotype specific amplification sequence 13 and a recognition sequence 14 complementary to a first segment 2 of the target nucleic acid sequence 1. The respective reverse selection primer 20A, 20B comprises, from a 5' end 21 to a 3' end 22, a second non-genotype specific amplification sequence 24 and a respective second locus recognition sequence 24A, 24B complementary to a respective sequence variant of the locus 9; 9B.

The first set of amplification primers 30, 40 comprises a forward amplification primer 30 and a reverse amplification primer 40. The forward amplification primer 30 is corresponding or complementary, preferably corresponding, to the first non-genotype specific amplification sequence 13 and the reverse amplification primer 40 is corresponding or complementary, preferably corresponding, to the second non-genotype specific amplification sequence 23.

The first set of amplification primers 30, 40 is present in a concentration of at least 100:1 with regard to a concentration of the *N* sets of selection primers 10A, 10B, 20; 10, 20A, 20B; 10A, 10B, 20A, 20B.

The method also comprises amplification and barcoding, in step S12, of the amplicons 4A, 4B using *N* barcoding primers 50A, 50B and a second set of amplification primers 30, 60; 40, 70 in a primer-dependent enzymatic reaction to yield barcoded amplicons 6A, 6B.

Each barcoding primer 50A, 50B of the *N* barcoding primers 50A, 50B comprises, in an embodiment and from a 5' end 51 to a 3' end 52, the first non-genotype specific amplification sequence 13 or a third non-genotype specific amplification sequence 53, a respective barcode sequence 55A, 55B and the respective first locus recognition sequence 14A, 14B or a respective third locus recognition sequence 54A, 54B complementary to the respective sequence variant of the locus 9; 9A. This embodiment comprises *N* so-called forward barcoding primers 50A, 50B.

In another embodiment, each barcoding primer 50A, 50B of the *N* barcoding primers 50A, 50B comprises, from a 5' end 51 to a 3' end 52, the second non-genotype specific amplification sequence 23 or a third non-genotype specific amplification sequence 53, a respective barcode sequence 55A, 55B and the respective second locus recognition sequence 24A, 24B or a respective fourth locus recognition sequence 56A, 56B complementary to the respective sequence variant of the locus 9; 9B. This embodiment comprises *N* so-called reverse barcoding primers 50A, 50B.

In a further embodiment, each forward barcoding primer of *N* forward barcoding primers comprises, in an embodiment and from a 5' end to a 3' end, the first non-genotype specific amplification sequence or a third non-genotype specific amplification sequence, a respective first barcode sequence and the respective first locus recognition sequence or a respective third locus recognition sequence complementary to the respective sequence variant of the locus. Furthermore, each reverse barcoding primer of *N* barcoding primers comprises, from a 5' end to a 3' end, the second non-genotype specific amplification sequence or a third non-genotype specific amplification sequence, a respective second barcode sequence and the respective second locus recognition sequence or a respective fourth locus recognition sequence complementary to the respective sequence variant of the locus.

The second set of amplification primers 30, 60; 40, 70 comprises a forward amplification primer 30, 60 and a reverse amplification primer 40, 70.

In the embodiment relating to using forward barcoding primers 50A, 50B the forward amplification primer 30, 60 of the second set of amplification primers 30, 60; 40, 70 is corresponding or complementary, preferably corresponding, to the first non-genotype specific amplification sequence 13 or the third non-genotype specific amplification sequence 53 and the reverse amplification primer 40 is corresponding or complementary, preferably corresponding, to the second non-genotype specific amplification sequence 23.

In the embodiment relating to using reverse barcoding primers 50A, 50B the forward amplification primer 30 of the second set of amplification primers 30, 60; 40, 70 is corresponding or complementary, preferably corresponding, to the first non-genotype specific amplification sequence 13 and the reverse amplification primer 40, 70 is corresponding or complementary, preferably corresponding, to the second non-genotype specific amplification sequence 23 or the third non-genotype specific amplification sequence 53.

The second set of amplification primers 30, 60; 40, 70 is present in a concentration of at least 100:1 with regard to a concentration of the N barcoding primers 50A, 50B.

The method also comprises genotyping the N sequence variants of the locus 9; 9A, 9B in step S13.

The genotyping in step S13 comprises, in an embodiment, contacting, during or after amplification and barcoding in step S12, each barcoded amplicon 6A, 6B with N labelled detection probes 90A, 90B comprising a respective label 92A, 92B and a respective sequence 91A, 91B complementary to a barcode sequence 55A, 55B of the N barcode sequences 55A, 55B. The genotyping also comprises, in this embodiment, relating an amount of detected label 92A, 92B to a specific genotype for the locus 9; 9A, 9B.

In another embodiment, the genotyping in step S13 comprises contacting, after amplification and barcoding in step S12, each barcoded amplicon 6A, 6B with a detection array of N genotype specific sequences. Each genotype specific sequence of the N genotype specific sequences is complementary to a respective barcode sequence 55A, 55B of the N barcode sequences 55A, 55B. The genotyping also comprises, in this another embodiment, relating detected hybridization of each barcoded amplicon 6A, 6B to the detection array to a specific genotype for the locus 9; 9A, 9B.

The comparatively much lower concentration and amount of the selection primers 10A, 10B, 20; 10, 20A, 20B; 10A, 10B, 20A, 20B and the barcoding primers 50A, 50B as compared to the amplification primers 30, 40; 60, 70 enables, as mentioned in the foregoing, high degree of multiplexing, i.e., usage of multiple sets of selection primers 10A, 10B, 20; 10, 20A, 20B; 10A, 10B, 20A, 20B and barcoding primers 50A, 50B in a single reaction container or vessel and still have low interaction between the primers. Accordingly, the selective amplification in step S11 and the amplification and barcoding in step S12 can be conducted in a single reaction container with a plurality of different sets of selection primers 10A, 10B, 20; 10, 20A, 20B; 10A, 10B, 20A, 20B and barcoding primers 50A, 50B.

For instance, the parameter N could be up to several thousands, including tens of thousands. It is, though, generally preferred if the parameter N is up to several hundreds, such as less than 1000. Hence, the parameter N is in an embodiment equal to or larger than 2, preferably equal to or larger than 5, more preferably equal to or larger than 10. The parameter *N* is also, or alternatively, equal to or smaller than 10000, preferably equal to or smaller than 1000, such as equal to or smaller than 500 or equal to or smaller than 100.

Each set of selection primers 10A, 10B, 20; 10, 20A, 20B; 10A, 10B, 20A, 20B of the N sets comprises at least one selection primer 10A, 10B; 20A, 20B that comprises a specific or unique sequence, i.e., the first or second locus recognition sequence 14A, 14B; 24A, 24B, and optionally one selection primer 20; 10 that is preferably common for all N sets. This means that the specific selection primer 10A, 10B; 20A, 20B can be used to interrogate the locus, whereas a same common selection primer 20; 10 can optionally be used for all sets.

In a preferred embodiment, the specific selection primer is the forward selection primer 10A, 10B. In such a case, the forward selection primer 10A, 10B comprises the specific or respective first locus recognition sequence 14A, 14B complementary to a respective sequence variant of the locus 9 and the first non-genotype specific amplification sequence 13 that is common for all forward selection primers 10A, 10B. In this preferred embodiment, the reverse selection primer is a common reverse selection primer 20 with the second non-genotype specific amplification sequence 23 and the recognition sequence 24 complementary to the second segment 3 of the target nucleic acid sequence 1A, 1B.

In another embodiment, the specific selection primer is the reverse selection primer 20A, 20B. In such a case, the reverse selection primer 20A, 20B comprises the specific or respective second locus recognition sequence 24A, 24B complementary to a respective sequence variant of the locus 9 and the second non-genotype specific amplification sequence 23 that is common for all reverse selection primers 20A, 20B. In this embodiment, the forward selection primer is a common forward selection primer 10 with the first non-genotype specific amplification sequence 13 and the recognition sequence 14 complementary to the first segment 2 of the target nucleic acid sequence 1A, 1B.

In a further embodiment, the specific selection primers are forward selection primer 10A, 10B and reverse selection primer 20A, 20B. In such a case, the forward selection primer 10A, 10B comprises a specific or respective first locus recognition sequence 14A, 14B complementary to a respective sequence variant of a first locus 9A and the first non-genotype specific amplification sequence 13 that is common for all forward selection primers 10A, 10B. The reverse selection primer 20A, 20B comprises the specific or respective second locus recognition sequence 24A, 24B complementary to a respective sequence variant of a second locus 9B and the second non-genotype specific amplification sequence 23 that is common for all reverse selection primers 20A, 20B.

In a preferred embodiment, the barcoding primers 50A, 50B are so-called forward barcoding primers 50A, 50B. In such an embodiment, the barcoding primers 50A, 50B comprise the first or third non-genotype specific amplification sequence 13, 53, the respective barcode sequence 55A, 55B and the respective first or third locus recognition sequence 14A, 14B; 54A, 54B.

In this preferred embodiment, the second set of amplification primers 30, 60; 40 comprises the forward amplification primer 30, 60 corresponding or complementary, preferably corresponding, to the first or third non-genotype specific amplification sequence 13, 53 and the reverse amplification primer 40 corresponding or complementary, preferably corresponding, to the second non-genotype specific amplification sequence 23.

In another embodiment, the barcoding primers 50A, 50B are so-called reverse barcoding primers 50A, 50B. In such an embodiment, the barcoding primers 50A, 50B comprise the second or third non-genotype specific amplification sequence 23, 53, the respective barcode sequence 55A, 55B and the respective second or fourth locus recognition sequence 24A, 24B; 56A, 56B.

In this embodiment, the second set of amplification primers 30; 40, 70 comprises the forward amplification primer 30 corresponding or complementary, preferably corresponding, to the first non-genotype specific amplification sequence 13 and the reverse amplification primer 40, 70 corresponding or complementary, preferably corresponding, to the second or third non-genotype specific amplification sequence 23, 53.

In a further embodiment, forward barcoding primers and reverse barcoding primers are used. In such an embodiment, the forward barcoding primers comprise the first or third non-genotype specific amplification sequence, the respective first barcode sequence 55A, 55B and the respective first or third locus recognition sequence and the reverse barcoding primers comprise the second or third non-genotype specific amplification sequence, the respective second barcode sequence and the respective second or fourth locus recognition sequence.

In this embodiment, the second set of amplification primers comprises the forward amplification primer corresponding or complementary, preferably corresponding, to the first or third non-genotype specific amplification sequence and the reverse amplification primer corresponding or complementary, preferably corresponding, to the second or third non-genotype specific amplification sequence

The third non-genotype specific amplification sequence 53 in a reverse barcoding primer(s) 50; 50A, 50B used in step S3 in Fig. 1 and step S12 in Fig. 6 could be the same or different as compared to the corresponding third non-genotype specific amplification sequence 53 in a forward barcoding primer 50; 50A, 50B.

In an embodiment, the method comprises an additional, optional pre-amplification step S10 as shown in Fig. 6. This step S10 basically corresponds to step S1 in Fig. 1. Hence, step S10 comprises pre-amplification of the target nucleic acid sequence 1A, 1B in a primer-dependent enzymatic reaction.

The pre-amplification in step S1 and S10 is, in an embodiment, Whole-Genome Amplification (WGA), such as random WGA. WGA is a method for robust amplification of an entire genome, or a portion thereof, starting from small quantities of DNA, typically in the ng range, and resulting in larger quantities of the amplified products, typically in the µg range.

There are different WGA techniques available, both Polymerase Chain Reaction (PCR) based, such as Degenerate Oligonucleotide PCR (DOP-PCR) and Primer Extension Preamplification (PEP), and non-PCR techniques, such as Multiple Displacement Amplification (MDA).

The main difference between DOP-PCR and PEP is that PEP utilizes random primers and low PCR annealing temperature, whereas DOP-PCR uses semi-degenerative primers and an increasing annealing temperature. MDA involves binding of random hexamers to denatured DNA followed by strand displacement synthesis, typically using the Phi 29 polymerase. Generally, MDA enables generation of longer DNA fragments without sequence bias as compared to the PCR-based techniques.

In an embodiment, the pre-amplification of step S1 and S10 comprises multiple displacement amplification of the target nucleic acid sequence 1A, 1B in a primer-dependent enzymatic reaction.

Sequence-Independent Amplification (SIA), including Sequence-Independent Single Primer Amplification (SISPA), enables unbiased detection of, among others, highly divergent or novel viruses. The SIA algorithm comprises nucleic acid amplification using various methods, including randomly primed PCR or restriction endonuclease digestion of the sample DNA and ligation of adaptor sequences, followed by PCR amplification. RNA can be analyzed with a reverse transcription step prior to amplification. Hence, SIA, such as SISPA, can be used as a pre-amplification technique in step S1 and S10.

Thus, in an embodiment, the pre-amplification of step S1 and S10 comprises sequence-independent amplification of the target nucleic acid sequence 1A, 1B in a primer-dependent enzymatic reaction.

The primer-dependent enzymatic reactions of steps S2-S3 and S11-S12 in Figs. 1 and 6 are preferably PCR reactions, preferably using a DNA polymerase, such as a heat-stable DNA polymerase, e.g., *Taq* polymerase.

The optional pre-amplification step S1 and S10 in Figs. 1 and 6 may also be PCR reactions, but could also include non-PCR reactions, such as MDA.

Figs. 8A, 9A and 10 schematically illustrate the optional pre-amplification and selective amplification of the method shown in Fig. 6.

In the optional pre-amplification, multiple original or starting nucleic acid molecules 8A-8C, such as in the form of multiple genomic sequences, are amplified, such as using random primers 80, 85 or sequence-independent amplification.

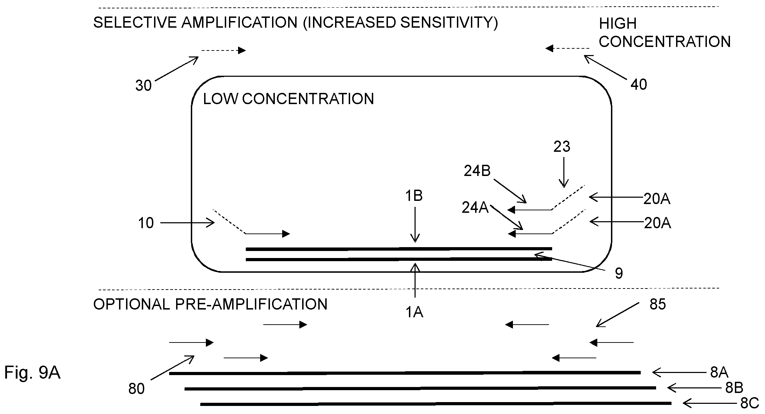

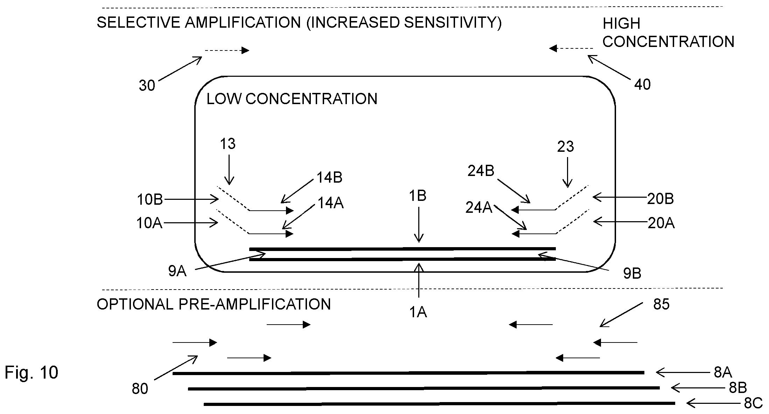

In the selective amplification, multiple sets of selection primers 10A, 10B, 20, see Fig. 8A; 10, 20A, 20B, see Fig. 9A; 10A, 10B, 20A, 20B, see Fig. 10, at low concentration and amplification primers 30, 40 at high concentration are added to amplify the target nucleic acid sequences 1A, 1B.

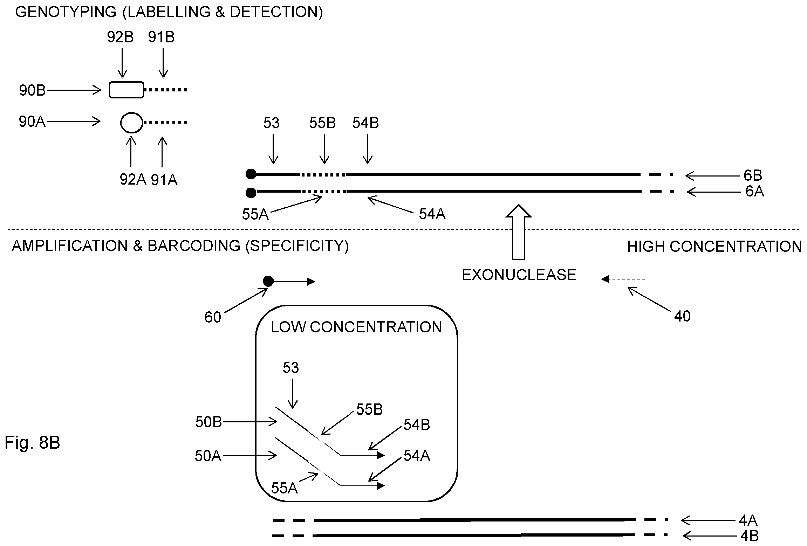

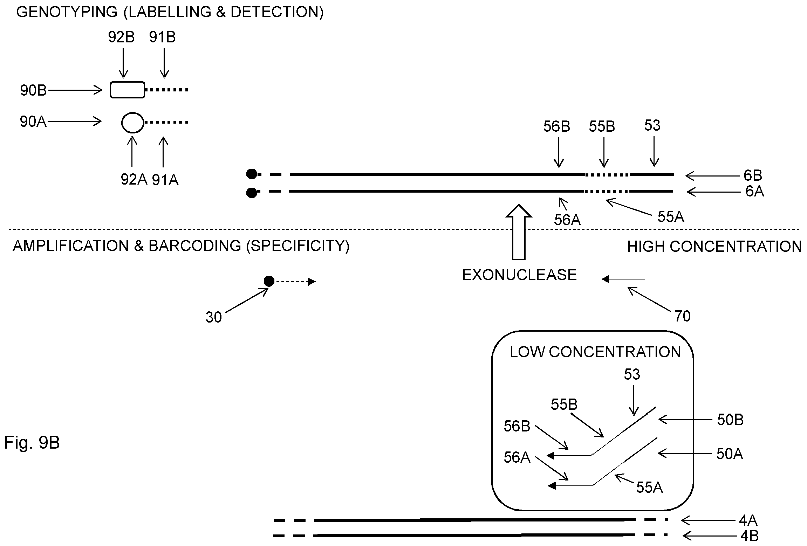

The resulting amplified amplicons 4A, 4B are shown in Figs. 8B and 9B additionally showing the amplification and barcoding and the genotyping. The amplification and barcoding is a primer-dependent enzymatic reaction using multiple barcoding primers 50A, 50B at low concentration and amplification primers 40, 60, see Fig. 8B, 30, 70, see Fig. 9B, at high concentration to form multiple barcoded amplicons 6A, 6B.

In an embodiment, the 5' end of the forward amplification primer 60 of the second set of amplification primers 30, 60; 40, 70 comprises a phosphate group represented by the black dot in Figs. 8B and 9B. In such an embodiment, the genotyping method comprises an additional step S20 as shown in Fig. 7. The method continues from step S12 in Fig. 6. The next step S20 comprises generating single-stranded barcoded amplicons by treating the barcoded amplicons 6A, 6B after step S12 with an exonuclease.

Exonucleases are enzymes that work by cleaving nucleotides one at a time from the end of a polynucleotide chain. A hydrolyzing reaction that breaks phosphodiester bonds at either the 5' end occurs.

In an embodiment, the exonuclease used in step S20 is an exonuclease that acts in the 5' to 3' direction catalyzing the removal of 5' nucleotides from double-stranded DNA (dsDNA). An example of such exonuclease that can be used according to the embodiments is exodeoxyribonuclease (lambda-induced) (EC 3.1.11.3), also referred to as lambda exonuclease, phage lambda-induced exonuclease, *Escherichia coli* exonuclease IV, exodeoxyribonuclease IV, or exonuclease IV.

The phosphate group at the 5' end of one of the strands of the double-stranded barcoded amplicons and introduced by the forward amplification primer 30, 60 forms a cap at the 5' end. The 5' capped strand thereby escapes enzymatic cleavage by the exonuclease. Accordingly, only the uncapped strand is degenerated in step S20, thereby resulting in single-stranded (ss) barcoded amplicons 6A, 6B.

Other techniques for obtaining single-stranded barcoded amplicons 6A, 6B could alternatively be used in step S20, such as denaturating the double-stranded barcoded amplicons.

Figs. 8B and 9B also illustrate an embodiment of the genotyping step S13 in Fig. 6. In this embodiment, each barcoded amplicon 6A, 6B, preferably in the form of single-stranded barcoded amplicons 6A, 6B, is contacted, during or after the amplification and barcoding step S12, with *N* labelled detection probes 90A, 90B. Each such detection probe 90A, 90B comprises a respective label 92A, 92B and a respective sequence 91A, 91B complementary to a barcode sequence 55A, 55B of the *N* barcode sequences 55A, 55B.

As a consequence, a detection probe 90A, 90B with a sequence 91A, 91B that is complementary to a barcode sequence 55A, 55B can thereby hybridize to the barcode sequence 55A, 55B and thereby to the barcoded amplicon 6A, 6B comprising this particular barcode sequence 55A, 55B. Each detection probe 90A, 90B has a respective label 92A, 92B enabling detection of the barcoded amplicon 6A, 6B once the detection probe 90A, 90B has bound to the barcoded amplicon 6A, 6B.

It is thereby possible to relate the amount of detected label 92A, 92B to a specific genotype for the locus 9.

In an embodiment, the genotyping step S13 in Fig. 6 comprises contacting, during or after amplification and barcoding in step S12, each barcoded amplicon 6A, 6B with a labelled common probe complementary to the first non-genotype specific amplification sequence 13 of the third non-genotype specific amplification sequence 53 and *N* labelled detection probes comprising a respective labelled bead and the respective sequence complementary to a barcode sequence 55A, 55B of the *N* barcode sequences 55A, 55B. In this embodiment, the genotyping step S13 also comprises relating an amount of detected labelled bead to the specific genotype for the locus 9.

This embodiment is particularly suitable for detection and reading with a LUMINEX^{®} system or machine.

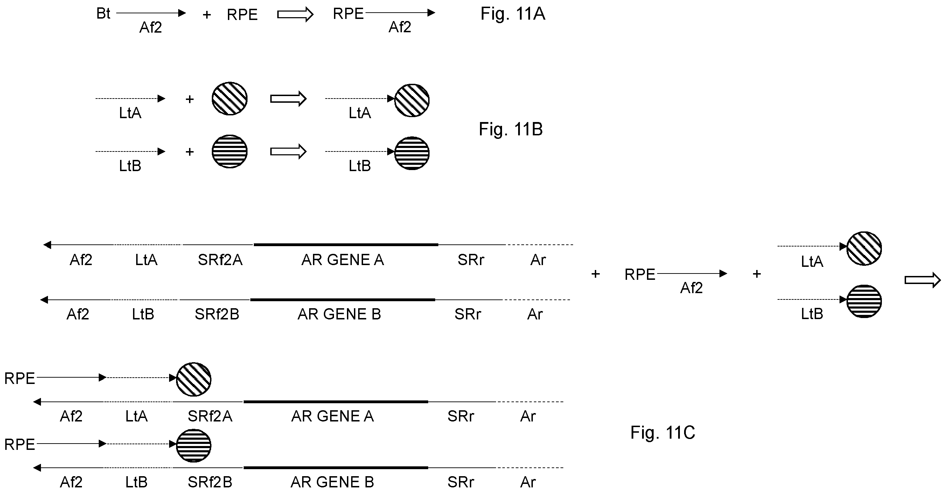

Figs. 11A to 11C schematically illustrate preparation of labelled common probes and *N* labelled detection probes and usage thereof in the genotyping.

Fig. 11A illustrate the preparation of the labelled common probe. In the illustrated example, the probe is complementary to the first or third non-genotype specific amplification sequence, denoted Af2 in the figures. The 5' end of the probe is biotinylated (Bt) to enable binding of a label, represented by R-phycoerythrin (RPE) in Fig. 11A. RPE is an intensely bright phycobiliporotein isolated from red algae and exhibits extremely bright red-orange fluorescence with high quantum yields. It is excited by light from 488 to 561 nm, with absorbance maxima at 496, 546 and 565 nm and a fluorescence emission peak at 578 nm. RPE should merely be seen as an illustrative example of label that can be used in the labelled common probe. Furthermore, biotinylation is an illustrative, but non-limiting, technique of binding the label, such as RPE, onto the probe sequence.

Fig. 11B illustrate the preparation of the *N* labelled detection probes according to an embodiment. In this embodiment, labelled beads, such as in the form of xMAP^{®} beads, are bound to the respective sequences, represented by LtA, LtB in the figure, complementary to the respective barcode sequences. In a particular embodiment, the labelled beads are bound to the 3' ends of the probe sequences. xMAP^{®} beads are color coded microspheres, which can be read and counted in a LUMINEX^{®} machine.

Fig. 11C illustrates the incubation or contacting of the barcoded amplicons with the labelled common probe and the *N* labelled detection probes. In Fig. 11C the barcoded amplicons comprise the first or third non-genotype specific sequence, denoted Af2, a barcode sequence, denoted LtA, LtB, the first or third recognition sequence, denoted SRf2A, SRf2B, the remaining sequence of the locus, denoted AR gene A, AR gene B, the recognition sequence, denoted SRr, and the second non-genotype specific amplification sequence, denoted Ar.

During the incubation or contacting step, the labelled common probe hybridizes to its complementary first or third non-genotype specific amplification sequence Af2. In addition, a labelled detection probe having a sequence complementary to a barcode sequence LtA, LtB hybridizes to the barcode sequence LtA, LtB in a barcoded amplicon. Accordingly, two labels are bound to and hybridized to the barcoded amplicons, i.e., the RPE and the xMAP^{®} bead.

In such a case, the LUMINEX^{®} machine can count the xMAP^{®} beads bound to barcoded amplicons together with a bound RPE-labelled common probe contributing to the fluorescence signal. When a sample is acquired, the fluorescence intensity (FI) observed for each color coded xMAP^{®} bead is recorded. Hence, a respective FI is acquired for each color coded xMAP^{®} bead, and thereby for each barcode sequence LtA, LtB, and each sequence variant of the locus.

More information of usage of the LUMINEX^{®} xMAP^{®} technology can be found in [4].

In another embodiment, the genotyping step S13 comprises contacting, during or after the amplification and barcoding in step S12, each barcoded amplicon 6A, 6B with a detection array of *N* genotype specific sequences. In this embodiment, each genotype specific sequence of the *N* genotype specific sequences is complementary to a respective barcode sequence 55A, 55B of the *N* barcode sequences 55A, 55B. The genotyping step also comprises, in this embodiment, relating detected hybridization of each barcoded amplicon 6A, 6B to the detection array to a specific genotype for the locus 9; 9A, 9B.

The detection array used in this embodiment can be in the form of a microarray, such as a microarray assay. More information of such microarrays can be found in [5-7].

Another aspect of the embodiments relates to a kit for amplification of a target nucleic acid sequence 1. The kit comprises a set of selection primers 10, 20, a first set of amplification primers 30, 40, a barcoding primer 50 and a second set of amplification primers 30, 60; 40, 70.

In an embodiment, the set of selection primers 10, 20 comprises a forward selection primer 10 and a reverse selection primer 20. The forward selection primer 10 comprises, from a 5' end 11 to a 3' end 12, a first non-genotype specific amplification sequence 13 and a first recognition sequence 14 complementary to a first segment 2 of the target nucleic acid sequence 1. The reverse selection primer 20 comprises, from a 5' end 21 to a 3' end 22, a second non-genotype specific amplification sequence 23 and a second recognition sequence 24 complementary to a second segment 3 of the target nucleic acid sequence 1.

In an embodiment, the first set of amplification primers 30, 40 comprises a forward amplification primer 30 and a reverse amplification primer 40. The forward amplification primer 30 is corresponding or complementary, preferably corresponding, to the first non-genotype specific amplification sequence 13 and the reverse amplification primer 40 corresponding or complementary, preferably corresponding, to the second non-genotype specific amplification sequence 23.

The first set of amplification primers 30, 40 is present in a concentration of at least 100:1 with regard to a concentration of the set of selection primers 10, 20.

In an embodiment, the second set of amplification primers 30, 60; 40, 70 comprises a forward amplification primer 30, 60 and a reverse amplification primer 40, 70.

In an embodiment, the barcoding primer 50 comprises, from a 5' end 51 to a 3' end 52, the first non-genotype specific amplification sequence 13 or a third non-genotype specific amplification sequence 53, a barcode sequence 55 and the first recognition sequence 14 or a third recognition sequence 54 complementary to a first segment 5 of the amplicon 4. In this embodiment, the forward amplification primer 30, 60 of the second set is corresponding or complementary, preferably corresponding, to the first non-genotype specific amplification sequence 13 or the third non-genotype specific amplification sequence 53 and the reverse amplification primer 40 is corresponding or complementary, preferably corresponding, to the second non-genotype specific amplification sequence 23.

In another embodiment, the barcoding primer 50 comprises, from the 5' end 51 to the 3' end 52, the second non-genotype specific amplification sequence 23 or a third non-genotype specific amplification sequence 53, a barcode sequence 55 and the second recognition sequence 24 or a fourth recognition sequence 56 complementary to a second segment 7 of the amplicon 4. In this embodiment, the forward amplification primer 30 of the second set is corresponding or complementary, preferably corresponding, to the first non-genotype specific amplification sequence 13 and the reverse amplification primer 40, 70 is corresponding or complementary, preferably corresponding, to the second non-genotype specific amplification sequence 23 or the third non-genotype specific amplification sequence 53.

In a further embodiment, a forward barcoding primer 50' and a reverse barcoding primer 50" are used. The forward barcoding primer 50' comprising, from a 5' end 51' to a 3' end 52', the first non-genotype specific amplification sequence 13 or a third non-genotype specific amplification sequence 53', a first barcode sequence 55' and the first recognition sequence 14 or a third recognition sequence 54 complementary to a first segment 5 of the amplicon 4. The reverse barcoding primer 50" comprises, from the 5' end 51" to the 3' end 52", the second non-genotype specific amplification sequence 23 or a third non-genotype specific amplification sequence 53", a second barcode sequence 55" and the second recognition sequence 24 or a fourth recognition sequence 56 complementary to a second segment 7 of the amplicon 4. In this embodiment, the forward amplification primer 30, 60 of the second set is corresponding or complementary, preferably corresponding, to the first non-genotype specific amplification sequence 13 or the third non-genotype specific amplification sequence 53' and the reverse amplification primer 40, 70 is corresponding or complementary, preferably corresponding, to the second non-genotype specific amplification sequence 23 or the third non-genotype specific amplification sequence 53".

The second set of amplification primers 30, 60; 40, 70 is present in a concentration of at least 100:1 with regard to a concentration of the barcoding primer 50.

If the forward amplification primer 30 has a nucleic acid sequence corresponding to the first non-genotype specific amplification sequence 13 and the reverse amplification primer 30 has a nucleic acid sequence corresponding to the second non-genotype specific amplification sequence 23 then the same amplification primers 30, 40 can be used in the second set as in the first set. However, in a particular embodiment, the 5' end of the forward amplification primer 30 used in the second set is phosphorylated, whereas there is preferably no phosphorylation of the 5' end of the forward amplification primer 30 in the first set. In such a particular embodiment, the same reverse amplification primer 40 can, though, be used in the first set and in the second set.

In an embodiment, the barcoding primer 50 comprises, from the 5' end 51 to the 3' end 52, the first non-genotype specific amplification sequence 13 or the third non-genotype specific amplification sequence 53, the barcode sequence 55 and the first recognition sequence 14 or the third recognition sequence 54. In this embodiment, the forward amplification primer 30, 60 of the second set is corresponding or complementary, preferably corresponding, to the first non-genotype specific amplification sequence 13 or the third non-genotype specific amplification sequence 53 and the reverse amplification primer 40 is corresponding or complementary, preferably corresponding, to the second non-genotype specific amplification sequence 23.

In a particular embodiment, the barcoding primer 50 comprises, from the 5' end 51 to the 3' end 52, the third non-genotype specific amplification sequence 53, the barcode sequence 55 and the third recognition sequence 54. In this particular embodiment, the forward amplification primer 60 of the second set is corresponding or complementary, preferably corresponding, to the third non-genotype specific amplification sequence 53 and the reverse amplification primer 40 is corresponding or complementary, preferably corresponding, to the second non-genotype specific amplification sequence 23.

In an embodiment, the forward selection primer 10 and the reverse selection primer 20 are present in a concentration of less than 100 nM, preferably of from 0.01 to 10 nM.

In an embodiment, the barcoding primer 50 is present in a concentration of less than 100 nM, preferably of from 0.01 to 10 nM.

In an embodiment, the first set of amplification primers 30, 40 is present in a concentration of from 100:1 to 100,000:1 with regard to the concentration of the set of selection primers 10, 20, such as from 100:1 to 10,000:1 with regard to the concentration of the set of selection primers 10, 20, e.g., from 500:1 to 5,000:1 with regard to the concentration of the set of selection primers 10, 20. In a particular embodiment, the first set of amplification primers 30, 40 is present in a concentration of about 1,000:1 with regard to the concentration of the set of selection primers 10, 20.

In an embodiment, the second set of amplification primers 30, 60; 40, 70 is present in a concentration of from 100:1 to 100,000:1 with regard to the concentration of the barcoding primer 50, such as from 100:1 to 10,000:1 with regard to the concentration of the barcoding primer 50, e.g., from 500:1 to 5,000:1 with regard to the concentration of the barcoding primer 50. In a particular embodiment, the second set of amplification primers 30, 60; 40, 70 is present in a concentration of about 1,000:1 with regard to the concentration of the barcoding primer 50.

A further aspect of the embodiments relates to a kit for genotyping a locus 9; 9A, 9B in a target nucleic acid sequence 1A, 1B. The locus 9; 9A, 9B has *N*≥2 sequence variants corresponding to *N* genotypes. The kit comprises *N* sets of selection primers 10A, 10B, 20; 10, 20A, 20B; 10A, 10B, 20A, 20B, a first set of amplification primers 30, 40, *N* barcoding primers 50A, 50B and a second set of amplification primers 30, 60; 40, 70. The kit preferably also comprises *N* labelled detection probes 90A, 90B or a detection array.

In an embodiment, each set of selection primers 10A, 10B, 20; 10, 20A, 20B; 10A, 10B, 20A, 20B of the *N* sets of selection primers 10A, 10B, 20; 10, 20A, 20B comprises i) a respective forward selection primer 10A, 10B and a common reverse selection primer 20, ii) a common forward selection primer 10 and a respective reverse selection primer 20A, 20B, or iii) a respective forward selection primer 10A, 10B and a respective reverse selection primer 20A, 20B.

The respective forward selection primer 10A, 10B comprises, from a 5' end 11 to a 3' end 12, a first non-genotype specific amplification sequence 13 and a respective first locus recognition sequence 14A, 14B complementary to a respective sequence variant of the locus 9; 9A. The common reverse selection primer 20 comprises, from a 5' end 21 to a 3' end 22, a second non-genotype specific amplification sequence 23 and a recognition sequence 24 complementary to a second segment 3 of the target nucleic acid sequence 1A, 1B. The common forward selection primer 10 comprises, from a 5' end 11 to a 3' end 12, a first non-genotype specific amplification sequence 13 and a recognition sequence 14 complementary to a first segment 2 of the target nucleic acid sequence 1A, 1B. The respective reverse selection primer 20A, 20B comprises, from a 5' end 21 to a 3' end 22, a second non-genotype specific amplification sequence 23 and a respective second locus recognition sequence 24A, 24B complementary to a respective sequence variant of the locus 9; 9B.

In an embodiment, the first set of amplification primers 30, 40 comprises a forward amplification primer 30 corresponding or complementary, preferably corresponding, to the first non-genotype specific amplification sequence 13 and a reverse amplification primer 40 corresponding or complementary, preferably corresponding, to the second non-genotype specific amplification sequence 23.

The first set of amplification primers 30, 40 is present in a concentration of at least 100:1 with regard to a concentration of the *N* sets of selection primers 10A, 10B, 20; 10, 20A, 20B; 10A, 10B, 20A, 20B.

The second set of amplification primers 30, 60; 40, 70 comprises a forward amplification primer 30, 60 and a reverse amplification primer 40, 70.

In an embodiment, each barcoding primer 50A, 50B of the N barcoding primers 50A, 50B comprises, from a 5' end 51 to a 3' end 52, the first non-genotype specific amplification sequence 13 or a third non-genotype specific amplification sequence 53, a respective barcode sequence 55A, 55B and the respective first locus recognition sequence 14A, 14B or a respective third locus recognition sequence 54A, 54B complementary to the respective sequence variant of the locus 9, 9A. In this embodiment, the forward amplification primer 30, 60 of the second set is corresponding or complementary, preferably corresponding, to the first non-genotype specific amplification sequence 13 or the third non-genotype specific amplification sequence 53 and the reverse amplification primer 40 corresponding or complementary, preferably corresponding, to the second non-genotype specific amplification sequence 23.

In another embodiment, each barcoding primer 50A, 50B of the *N* barcoding primers 50A, 50B comprises, from the 5' end 51 to the 3' end 52, the second non-genotype specific amplification sequence 23 or a third non-genotype specific amplification sequence 53, a respective barcode sequence 55A, 55B and the respective second locus recognition sequence 24A, 24B or a respective fourth locus recognition sequence 56A, 56B complementary to the respective sequence variant of the locus 9, 9B. In this embodiment, the forward amplification primer 30 of the second set is corresponding or complementary, preferably corresponding, to the first non-genotype specific amplification sequence 13 and the reverse amplification primer 40, 70 corresponding or complementary, preferably corresponding, to the second non-genotype specific amplification sequence 23 or the third non-genotype specific amplification sequence 53.

It is also possible to use a combination of *N* forward barcoding primers and *N* reverse barcoding primers as previously disclosed herein.

The second set of amplification primers 30, 60; 40, 70 is present in a concentration of at least 100:1 with regard to a concentration of the *N* barcoding primers 50A, 50B.

The *N* labelled detection probes 90A, 90B comprise a respective label 92A, 92B and a respective sequence 91A, 91B complementary to a barcode sequence 55A, 55B of the *N* barcode sequences 55A, 55B.

The detection array is a detection array of *N* genotype specific sequences. Each genotype specific sequence of the *N* genotype specific sequences is complementary to a respective barcode sequence 55A, 55B of the *N* barcode sequences 55A, 55B.

If the forward amplification primer 30 has a nucleic acid sequence corresponding or complementary, preferably corresponding, to the first non-genotype specific amplification sequence 13 and the reverse amplification primer 30 has a nucleic acid sequence corresponding or complementary, preferably corresponding, to the second non-genotype specific amplification sequence 23 then the same amplification primers 30, 40 can be used in the second set as in the first set. However, in a particular embodiment the 5' end of the forward amplification primer 30 used in the second set is phosphorylated, whereas there is preferably no phosphorylation of the 5' end of the forward amplification primer 30 in the first set. In such a particular embodiment, the same reverse amplification primer 40 can, though, be used in the first set and in the second set.

In an embodiment, each set of selection primers 10A, 10B, 20; 10, 20A, 20B; 10A, 10B, 20A, 20B of the *N* sets of selection primers 10A, 10B, 20; 10, 20A, 20B; 10A, 10B, 20A, 20B comprises the respective forward selection primer 10A, 10B and the common reverse selection primer 20. In this embodiment, the respective forward selection primer 10A, 10B comprises, from the 5' end 11 to the 3' end 12, the first non-genotype specific amplification sequence 13 and the respective first locus recognition sequence 14A, 14B complementary to the respective sequence variant of the locus 9. The common reverse selection primer 20 comprising, from the 5' end 21 to the 3' end 22, the second non-genotype specific amplification sequence 23 and the recognition sequence 24 complementary to the second segment 3 of the target nucleic acid sequence 1A, 1B. In this embodiment, each barcoding primer 50A, 50B of the *N* barcoding primers 50A, 50B comprises, from the 5' end 51 to the 3' end 52, the first non-genotype specific amplification sequence 13 or the third non-genotype specific amplification sequence 53, the respective barcode sequence 55A, 55B and the respective first locus recognition sequence 14A, 14B or the respective third locus recognition sequence 54A, 54B. In this embodiment, the forward amplification primer 30, 60 of the second set is corresponding or complementary, preferably corresponding, to the first non-genotype specific amplification sequence 13 or the third non-genotype specific amplification sequence 53 and the reverse amplification primer 40 is corresponding or complementary, preferably corresponding, to the second non-genotype specific amplification sequence 23.

In a particular embodiment, each barcoding primer 50A, 50B of the *N* barcoding primers 50A, 50B comprises, from the 5' end 51 to the 3' end 52, the third non-genotype specific amplification sequence 53A, 53B, the respective barcode sequence 55A, 55B and the respective third locus recognition sequence 54A, 54B. In this particular embodiment, the forward amplification primer 60 of the second set is corresponding or complementary, preferably corresponding, to the third non-genotype specific amplification sequence 53 and the reverse amplification primer 40 corresponding or complementary, preferably corresponding, to the second non-genotype specific amplification sequence 23.

In another embodiment, each set of selection primers 10A, 10B, 20; 10, 20A, 20B; 10A, 10B, 20A, 20B of the *N* sets of selection primers 10A, 10B, 20; 10, 20A, 20B; 10A, 10B, 20A, 20B comprises the common forward selection primer 10 and the respective reverse selection primer 20A, 20B. In this embodiment, the common forward selection primer 10 comprises, from the 5' end 11 to the 3' end 12, the first non-genotype specific amplification sequence 13 and the recognition sequence 14 complementary to the first segment 2 of the target nucleic acid sequence 1A, 1B. The respective reverse selection primer 20A, 20B comprises, from the 5' end 21 to the 3' end 22, the second non-genotype specific amplification sequence 23 and the respective second locus recognition sequence 24A, 24B complementary to the respective sequence variant of the locus 9. In this embodiment, each barcoding primer 50A, 50B of the *N* barcoding primers 50A, 50B comprises, from the 5' end 51 to the 3' end 52, the second non-genotype specific amplification sequence 23 or the third non-genotype specific amplification sequence 53, the respective barcode sequence 55A, 55B and the respective second locus recognition sequence 24A, 24B or the respective fourth locus recognition sequence 56A, 56B. In this embodiment, the forward amplification primer 30 of the second set is corresponding or complementary, preferably corresponding, to the first non-genotype specific amplification sequence 13 and the reverse amplification primer 70 is corresponding or complementary, preferably corresponding, to the second non-genotype specific amplification sequence 23 or the third non-genotype specific amplification sequence 53.

In a further embodiment, each set of selection primers 10A, 10B, 20; 10, 20A, 20B; 10A, 10B, 20A, 20B of the *N* sets of selection primers 10A, 10B, 20; 10, 20A, 20B; 10A, 10B, 20A, 20B comprises the respective forward selection primer 10A, 10B and the respective reverse selection primer 20A, 20B. In this embodiment, the respective forward selection primer 10A, 10B comprises, from the 5' end 11 to the 3' end 12, the first non-genotype specific amplification sequence 13 and the respective first locus recognition sequence 14A, 14B complementary to the respective sequence variant of the first locus 9A. The respective reverse selection primer 20A, 20B comprises, from the 5' end 21 to the 3' end 22, the second non-genotype specific amplification sequence 23 and the respective second locus recognition sequence 24A, 24B complementary to the respective sequence variant of the locus 9B.

In an embodiment, the *N* labelled detection probes comprises a respective labelled bead and the respective sequence complementary to a barcode sequence 55A, 55B of the *N* barcode sequences 55A, 55B. In this embodiment, the kit further comprises a labelled common probe complementary to the first non-genotype specific amplification sequence 13 or the third non-genotype specific amplification sequence 53.

In an embodiment, i) each respective forward selection primer 10A, 10B and the common reverse selection primer 20, ii) the common forward selection primer 10 and each respective reverse selection primer 20A, 20B, or iii) each respective forward selection primer 10A, 10B and each respective reverse selection primer 20A, 20B is present in a concentration of less than 100 nM, preferably of from 0.01 to 10 nM.

In an embodiment, each barcoding primer 50A, 50B is present in a concentration of less than 100 nM, preferably of from 0.01 to 10 nM.

In an embodiment, the first set of amplification primers 30, 40 is present in a concentration of from 100:1 to 100,000:1 with regard to the concentration of the *N* sets of selection primers 10A, 10B, 20; 10, 20A, 20B; 10A, 10B, 20A, 20B, such as from 100:1 to 10,000:1 with regard to the concentration of the *N* sets of selection primers 10A, 10B, 20; 10, 20A, 20B; 10A, 10B, 20A, 20B, e.g., from 500:1 to 5,000:1 with regard to the concentration of the *N* sets of selection primers 10A, 10B, 20; 10, 20A, 20B; 10A, 10B, 20A, 20B. In a particular embodiment, the first set of amplification primers 30, 40 is present in a concentration of about 1,000:1 with regard to the concentration of the *N* sets of selection primers 10A, 10B, 20; 10, 20A, 20B; 10A, 10B, 20A, 20B.

In an embodiment, the second set of amplification primers 30, 60; 40, 70 is present in a concentration of from 100:1 to 100,000:1 with regard to the concentration of the *N* barcoding primers 50A, 50B, such as from 100:1 to 10,000:1 with regard to the concentration of the *N* barcoding primers 50A, 50B, e.g., from 500:1 to 5,000:1 with regard to the concentration of the *N* barcoding primers 50A, 50B. In a particular embodiment, the second set of amplification primers 30, 60; 40, 70 is present in a concentration of about 1,000:1 with regard to the concentration of the *N* barcoding primers 50A, 50B.

In an embodiment, a 5' end of the forward amplification primer 30, 60 of the second set of amplification primers 30, 60; 40, 70 comprises a phosphate group.

The methods and kits of the embodiments can be using in various applications including, but not limited to, SNP genotyping, genetic disease screening, gene expression profiling, Human Leukocyte Antigen (HLA) DNA typing, microbial detection, pathotyping, antibiotic resistance profiling, etc.

### EXAMPLE

The present example discloses the development of a rapid and flexible multiplex assay for culture-independent detection of antibiotic resistance genes that can be used for molecular diagnostics and epidemiology studies. The development of antibiotic resistance among harmful bacteria has greatly been increased by the excessive use and abuse of antibiotics in both human and veterinary medicine. It is now recognized as a very serious global health threat, which could result in a return to the pre-antibiotic era, as warned by the WHO. Rapid and accurate determination of antibiotic resistance is essential for effective treatment and infection control. However, traditional methods for antibiotic susceptibility testing of bacterial pathogens are cultivation based and require at least 24 hours of incubation. In addition, since resistance genes are known to aggregate upon plasmids, horizontal gene transfer can readily confer multi-resistance between even distantly related bacteria. An implication of this is that it might not be possible to determine the complete resistance profile of bacterial pathogens by using conventional molecular methods for rapid detection, such as real time PCR, because of their limited multiplexing capacity. In responds to this, a novel PCR-based approach has been developed.

### Material and Methods

### General assay design

A two-step PCR-based assay was designed to allow rapid multiplex detection of selected antibiotic resistance genes using LUMINEX^{®} xMAP^{®} Technology. First, amplicons are generated for the target regions using selection primers, each consisting of a gene-specific sequence and a common amplification tag, in combination with corresponding amplification primers. By keeping a significantly lowered concentrations of the selection primers (e.g., 1,000 times lower) but normal concentrations of their common forward and reverse amplification primers, primer interactions are strongly reduced, which permits a high degree of multiplexing. In the second step, target regions within the products from the first step are amplified using barcoding-primers, each consisting of a target-specific sequence, a FlexMAP^{®} tag (Luminex Corporation) and a common amplification tag, in combination with a second set of common amplification primers, of which the forward primer is 5'-phosphorylated. As in the previous step, the barcoding-primers and the common amplification primers are kept at lowered and normal concentrations, respectively. Lambda exonuclease digestion of the 5' phosphorylated strand was used to render the barcoded amplicons single-stranded. Prior to LUMINEX^{®} analysis, the single stranded amplicons were hybridized to color-coded microbeads with anti-tag sequences and fluorescently labelled probes, consisting of a shorter (18 nt) and 5' biotinylated version of the common forward amplification primer coupled with streptavidin R-phycoerythrin (SAPE).

### Plasmids and bacterial strains

The pUUH239.2 plasmid was used to develop and optimize the assay. This plasmid was previous characterized by Sandegren *et al.* [8] and encodes resistance to β-lactams (*bla*_{CTX-M-15}, *bla*_{TEM-1} and *bla*_{OXA-1}), aminoglycosides *[aac-(6)-1b-cr* and *aadA2*], tetracyclines [tet(A) and tetR], trimethoprim (*dhfrXII*), sulphonamides (*sul1*), quaternary ammonium compounds (*qacEΔ1*), macrolides *[mph(A)-mxr-mphR(A)]* and heavy metal ions (silver, copper and arsenic). To evaluate the performance of the assay on bacterial samples, six strains were kindly provided by Dr. Linus Sandegren. As listed in Table 1, four of these strains carried modified versions the pUUH239.2 plasmid, one carried the original pUUH239.2 plasmid, and one carried a largely uncharacterized plasmid with the New Delhi Metallo-beta lactamase (NDM-1) resistance gene.

**Table 1 - Bacterial strains**

| **Name** | **Bacteria** | **Plasmid** | **Modification** |
|---|---|---|---|
| DA15001 | *Escherichia coli* | pUUH239.2 | wild type |
| DA14758 | *E. coli* | pUUH239.2 | deletion of *chrA, sul1, aadA2, dhfrXII* |
| DA14792 | *E. coli* | pUUH239.2 | deletion of *tet(A), tetR* |
| DA14805 | *E. coli* | pUUH239.2 | deletion of *mph(A)-mxr-mphR(A), chrA, sul1, aadA2, dhfrXII* |
| DA15832 | *E. coli* | pUUH239.2 | deletion av *mph(A)-mxr-mphR(A)* |
| DA14833 | *E. coli* | pUUH239.2 | 3 copies of *aac(6')-1b-cr*, *bla*_{OXA-1}, tet(A), and *tetR* |
| DA28170 | *Klebisella pneumoniae* | Uncharacterized plasmid | Encodes NDM-1 (other resistance genes unknown) |

### PCR primer and probe design

The 11 antibiotic resistance genes selected for the multiplex assay are listed in Table 2. Of these genes, all except NDM-1 were present in the pUUH239.2 plasmid. To ensure a broad detection range, different variants of the selected resistance genes were retrieved from GenBank (http://www.ncbi.nlm.nih.gov). For each gene, the sequences were aligned using the MUSCLE software with its default setting followed by manual adjustment. Gene specific primer sequences were designed for conserved regions within the aligned sequences using the Primer 3 plus online tool with an optimal primer size of 20 bases and annealing temperature of 60°C. When needed, degenerate nucleotides were manually incorporated for improved detection of sequence variants. Selected 24-base FlexMAP^{®} TAGs were used as barcodes to enable multiplex detection using LUMINEX^{®} FlexMAP^{®} microspheres pre-coupled with capture oligonucleotides (anti-TAGs). Three FlexMAP^{®} TAGs were also chosen to serve as general amplification tags and corresponding primers. The resulting primer constructs and probes are listed in Table 3 and were synthesized by Sigma-Aldrich.

**Table 2 - Selected antibiotic resistance genes**

| **Gene** | **Abbreviated designation** | **Function** |
|---|---|---|
| *mrx* | mrx | Macrolide resistance |
| *mphR(A)* | mph | Macrolide resistance, repressor protein |
| *sul1* | sul | Dihydropteroate synthase, sulfonamide resistance |
| *aadA2* | aad | Aminoglycoside resistance |
| *dhfrXII* | dhf | Dihydrofolate reductase |
| *bla_{TEM-1}* | blaT | Beta-lactamase |
| *bla_{CTX-M-15}* | blaC | Extended spectrum beta-lactamase |
| *aac-(6')-1b-cr* | aac | Aminoglycoside/quinolone resistance |
| *bla_{OXA-1}* | blaO | β-lactamase |
| *tet(A)* | tet | Tetracycline resistance |
| NDM-1 | ndm | Metallo-beta-lactamase with nearly complete resistance to all beta-lactam antibiotics. |

| **Table 3** - Primer and probe sequences | | | | | |
|---|---|---|---|---|---|
| **Name** | **Target** | **Description** | **D*** | **Sequence** | **SEQ ID NO:** |
| [Phos]Fa023AMP | Common | High conc. selective amplification primer | F | [Phos]TTCAATCATTCAAAT CTCAACTTT | 1 |
| [Phos]Fa035 | Common | High conc. detection amplification primer | F | [Phos]CAATTTCATCATTCA TTCATTTCA | 2 |
| Ra051 | Common | High conc. universal reverse primer | R | [Phos]TTCAATCATTCAAAT CTCAACTTT [Phos]CAATTTCATCATTCA TTCATTTCA TCATTTCAATCAATCATCA ACAAT [Btn]CAATTTCATCATTCAT TC | 3 |
| [Btn]Fa035(D18) | Common | Probe for RPE-labelling | F | [Btn]CAATTTCATCATTCAT TC | 4 |
| Fa023bAMPaad | aad | Selective amplification of target sequence | F | TTCAATCATTCAAATCTCA ACTTTCGTTGCTGGCCGT RCATTTG | 5 |
| Fa035b031aad | aad amplicon | Gene specific barcoding of amplicon | F | TTCAATCATTCAAATCTCA ACTTTCGTTGCTGGCCGT RCATTTG CAATTTCATCATTCATTCA TTTCATTCACTTTTCAATCA ACTTTAATCCATTTGTACG GCTCCGCAGT TCATTTCAATCAATCATCA ACAATGTCATTGCGCTGC CATTCTC TTCACTTTTCAATCAACTTT AATC[AmC7] | 6 |
| Ra051aad | aad | Introduction of universal reverse sequence | R | TCATTTCAATCAATCATCA ACAATGTCATTGCGCTGC CATTCTC | 7 |
| ab031 [AmC7] | Barcoded aad amplicon | Capture probe for LUMINEX^{®} bead | | TTCACTTTTCAATCAACTTT AATC[AmC7] | 8 |
| Fa023AMPacc | acc | Selective amplification of target sequence | F | TTCAATCATTCAAATCTCA ACTTTAGAAGAAGCACGC CCGACAC CAATTTCATCATTCATTCA | 9 |
| Fa035b046acc | acc | Gene specific barcoding | F | CAATTTCATCATTCATTCA | 10 |
| | amplicon | of amplicon | | TTTCATACATCAACAATTC ATTCAATACACGCAAGAG TCCGTCACTCCA | |
| Ra051acc | acc | Introduction of universal reverse sequence | R | TTTCATACATCAACAATTC ATTCAATACACGCAAGAG TCCGTCACTCCA TCATTTCAATCAATCATCA ACAATGCCCAGTTGTGAT GCATTCG TACATCAACAATTCATTCA ATACA[AmC7] | 11 |
| ab046[AmC7] | Barcoded acc amplicon | Capture probe for LUMINEX^{®} bead | | TACATCAACAATTCATTCA ATACA[AmC7] | 12 |
| Fa023AMPblaC2 | blaC | Selective amplification of target sequence | F | TTCAATCATTCAAATCTCA ACTTTGCAGACTGGGTGT GGCATTG | 13 |
| Fa035b071blaC2 | blaC amplicon | Gene specific barcoding of amplicon | F | TTCAATCATTCAAATCTCA ACTTTGCAGACTGGGTGT GGCATTG CAATTTCATCATTCATTCA TTTCAATCATTACAATCCA ATCAATTCATTGCGATGTG CAGCACCAGTA TCATTTCAATCAATCATCA ACAATTGACGTGCTTTTCC GCAATC ATCATTACAATCCAATCAA TTCAT[AmC7] | 14 |
| Ra051blaC2 | blaC | Introduction of universal reverse sequence | R | TCATTTCAATCAATCATCA ACAATTGACGTGCTTTTCC GCAATC | 15 |
| ab071 [AmC7] | Barcoded blaC amplicon | Capture probe for LUMINEX^{®} bead | | ATCATTACAATCCAATCAA TTCAT[AmC7] | 16 |
| Fa023AMPdhf | dhf | Selective amplification of target sequence | F | TTCAATCATTCAAATCTCA ACTTTSATGGGWGCCAAT CGGGTTA | 17 |
| Fa035b005dhf | dhf amplicon | Gene specific barcoding of amplicon | F | TTCAATCATTCAAATCTCA ACTTTSATGGGWGCCAAT CGGGTTA CAATTTCATCATTCATTCA TTTCACAATTCAAATCACA ATAATCAATCCAATCGGGT TATTGGCAATGG TCATTTCAATCAATCATCA ACAATAGGCGTCACCCTC GAAGGT CAATTCAAATCACAATAAT CAATC[AmC7] | 18 |
| Ra051 dhf | dhf | Introduction of universal reverse sequence | R | TCATTTCAATCAATCATCA ACAATAGGCGTCACCCTC GAAGGT | 19 |
| ab005[AmC7] | Barcoded dhf | Capture probe for LUMINEX^{®} bead | | CAATTCAAATCACAATAAT CAATC[AmC7] | 20 |
| | amplicon | | | | |
| Fa023AMPmph | mph | Selective amplification of target sequence | F | TTCAATCATTCAAATCTCA ACTTTTACGCATGTGCCT GGAGGAG | 21 |
| Fa035b016mph | mph amplicon | Gene specific barcoding of amplicon | F | TTCAATCATTCAAATCTCA ACTTTTACGCATGTGCCT GGAGGAG CAATTTCATCATTCATTCA TTTCAAATCAATCTTCATT CAAATCATCAGGTTCCGCT GGATCGCAAG TCATTTCAATCAATCATCA ACAATGCTCGTTCGGAGC ATGAACA AATCAATCTTCATTCAAAT CATCA[AmC7] | 22 |
| Ra051mph | mph | Introduction of universal reverse sequence | R | TCATTTCAATCAATCATCA ACAATGCTCGTTCGGAGC ATGAACA | 23 |
| ab016[AmC7] | Barcoded mph amplicon | Capture probe for LUMINEX^{®} bead | | AATCAATCTTCATTCAAAT CATCA[AmC7] | 24 |
| Fa023AMPmrx | mrx | Selective amplification of target sequence | F | TTCAATCATTCAAATCTCA ACTTTGCCTGGCAACTGT GAAAGCA | 25 |
| Fa035b015mrx | mrx amplicon | Gene specific barcoding of amplicon | F | TTCAATCATTCAAATCTCA ACTTTGCCTGGCAACTGT GAAAGCA CAATTTCATCATTCATTCA TTTCAATACTTCATTCATTC ATCAATTCACACCGCAGA GCCACAAAATG TCATTTCAATCAATCATCA ACAATGCTGCGCCCTCTC TTTGGTA ATACTTCATTCATTCATCA ATTCA[AmC7] | 26 |
| Ra051mrx | mrx | Introduction of universal reverse sequence | R | TCATTTCAATCAATCATCA ACAATGCTGCGCCCTCTC TTTGGTA | 27 |
| ab015[AmC7] | Barcoded mrx amplicon | Capture probe for LUMINEX^{®} bead | | ATACTTCATTCATTCATCA ATTCA[AmC7] | 28 |
| Fa023AMPndm | ndm | Selective amplification of target sequence | F | TTCAATCATTCAAATCTCA ACTTTCGCCCAGATCCTC AACTGGA | 29 |
| Fa035b022ndm | ndm amplicon | Gene specific barcoding of amplicon | F | TTCAATCATTCAAATCTCA ACTTTCGCCCAGATCCTC AACTGGA CAATTTCATCATTCATTCA TTTCAAATCCTTTTTACTCA ATTCAATCAGGACAAGAT GGGCGGTATGG | 30 |
| Ra051ndm | ndm | Introduction of universal reverse sequence | R | TCATTTCAATCAATCATCA ACAATGGGGCCGGGGTAA AATACCT | 31 |
| ab022[AmC7] | Barcoded ndm amplicon | Capture probe for LUMINEX^{®} bead | | TCATTTCAATCAATCATCA ACAATGGGGCCGGGGTAA AATACCT AATCCTTTTTACTCAATTC AATCA[AmC7] | 32 |
| Fa023AMPsul | sul | Selective amplification of target sequence | F | TTCAATCATTCAAATCTCA ACTTTTCCGATCAGATGC ACCGTGT | 33 |
| Fa035b021sul | sul amplicon | Gene specific barcoding of amplicon | F | TTCAATCATTCAAATCTCA ACTTTTCCGATCAGATGC ACCGTGT CAATTTCATCATTCATTCA TTTCAAATCCTTTCTTTAAT CTCAAATCACGACGAGAT TGTGCGGTTCTT TCATTTCAATCAATCATCA ACAATACACCGAGACCAA TAGCGGAAG AATCCTTTCTTTAATCTCA AATCA[AmC7] | 34 |
| Ra051sul | sul | Introduction of universal reverse sequence | R | TCATTTCAATCAATCATCA ACAATACACCGAGACCAA TAGCGGAAG | 35 |
| ab021 [AmC7] | Barcoded sul amplicon | Capture probe for LUMINEX^{®} bead | | AATCCTTTCTTTAATCTCA AATCA[AmC7] | 36 |
| Fa023AMPtetA | tetA | Selective amplification of target sequence | F | TTCAATCATTCAAATCTCA ACTTTCGACGGCRCAGGC TACATCC | 37 |
| Fa035b011tetA | tetA amplicon | Gene specific barcoding of amplicon | F | TTCAATCATTCAAATCTCA ACTTTCGACGGCRCAGGC TACATCC CAATTTCATCATTCATTCA TTTCATACAAATCATCAAT CACTTTAATCTCCCGATYA TGGTCCTGCTT TCATTTCAATCAATCATCA ACAATCGCATAGATCGCC GTGAAGA TACAAATCATCAATCACTT TAATC[AmC7] | 38 |
| Ra051tetA | tetA | Introduction of universal reverse sequence | R | TCATTTCAATCAATCATCA ACAATCGCATAGATCGCC GTGAAGA | 39 |
| ab011 [AmC7] | Barcoded tetA amplicon | Capture probe for LUMINEX^{®} bead | | TACAAATCATCAATCACTT TAATC[AmC7] | 40 |
| Fa023AMPblaO | blaO | Selective amplification of target sequence | F | TTCAATCATTCAAATCTCA ACTTTTCCACAAACGCTG | 41 |
| | | | | AAATTGCTC | |
| Fa035b009blaO | blaO amplicon | Gene specific barcoding of amplicon | F | CAATTTCATCATTCATTCA TTTCATAATCTTCTATATCA ACATCTTACACACCAAAG ACGTGGATGCAA | 42 |
| Ra051blaO | blaO | Introduction of universal reverse sequence | R | AAATTGCTC CAATTTCATCATTCATTCA TTTCATAATCTTCTATATCA ACATCTTACACACCAAAG ACGTGGATGCAA TCATTTCAATCAATCATCA ACAATTTAAGCTACTTTCG AGCCATGCTTC TAATCTTCTATATCAACAT CTTAC[AmC7] | 43 |
| ab009[AmC7] | Barcoded blaO amplicon | Capture probe for LUMINEX^{®} bead | | TAATCTTCTATATCAACAT CTTAC[AmC7] | 44 |
| Fa023AMPblaT | blaT | Selective amplification of target sequence | F | TTCAATCATTCAAATCTCA ACTTTGCTCACCCAGAAA CGCTGGT | 45 |
| Fa035b073blaT | blaT amplicon | Gene specific barcoding of amplicon | F | TTCAATCATTCAAATCTCA ACTTTGCTCACCCAGAAA CGCTGGT CAATTTCATCATTCATTCA TTTCAATCAAATCTCATCA ATTCAACAATGCATCTTAC GGATGGCATGA TCATTTCAATCAATCATCA ACAATTTCGGTCCTCCGAT CGTTGT ATCAAATCTCATCAATTCA ACAAT[AmC7] | 46 |
| Ra051 blaT | blaT | Introduction of universal reverse sequence | R | TCATTTCAATCAATCATCA ACAATTTCGGTCCTCCGAT CGTTGT | 47 |
| ab073[AmC7] | Barcoded blaT amplicon | Capture probe for LUMINEX^{®} bead | | ATCAAATCTCATCAATTCA ACAAT[AmC7] | 48 |

Grey highlight = common forward sequence for amplification;
Bold = gene specific;
Italic = gene-specific barcode-sequence for LUMINEX^{®} detection;
Underline = common forward sequence for amplification and barcoding of amplicons
D* = direction, F = Forward and R = Reverse
[Phos] = phosphorylated
[Btn] = biotinylated
[AmC7] = Amino Linker C7 (C7-NH₂)

### Multiplexed PCR amplification

The first round of PCR was performed in 25-µl reaction volumes containing 2.5 µl 10× PCR buffer (Applied Biosystems), 3 µl MgCl₂ (25 mM), 0.5 µl dNTPs (each 10mM), 3 µl primer mix (a mixture of all selection primers, each at a concentration of 100 nM), 1 µl of each amplification primer ([Phos]Fa023AMP and Ra051, both at 10 µM), 0.5 µL Platinum Taq polymerase (Applied Biosystems), 2 µl template DNA, and 12.5 µL H₂O. Amplification was carried out with an initial denaturation at 95 °C for 3 min, followed by 40 cycles of denaturation at 95 °C for 10 s, annealing at 55 °C for 15 s and extension at 72 °C for 20 s, and ended with a final extension at 72 °C for 15 min. The second PCR round was performed essentially as above, except that 1 µl of the product from the first step was used as template, the forward amplification primer was changed (to [Phos]Fa035), and the number of amplification cycles was reduced to 30. The final amplification products were subjected to Lambda exonuclease digestion to generate single-stranded DNA (ssDNA) targets. Each digestion reaction was carried out in a total volume of 15 µl using 13.2 µl of PCR product, 1.5 µl of 10× Lambda buffer, and 3 units of Lambda exonuclease (Thermo Fisher Scientific, USA). Reaction mixtures were incubated at 37 °C for 30 min, followed by a heat inactivation step at 80 °C for 15 min according to the manufacturer's instructions.

### Preparation of microbeads with anti-tag sequences

Anti-tag sequences containing a 3'-amino group with a seven-carbon spacer were conjugated to XMAP^{®} color-coded carboxylated microspheres (Luminex; Austin, TX) as follows. For each anti-tag, 5.0 × 10⁶ microspheres (suspended according to the manufacturer's instructions) were pelleted at 8,000 × g for 2 min and resuspended in 50 µl of 0.1 M 2-morpholinoethane sulfonic acid (MES) (pH 4.5) before the addition of 1 µl (0.1 nmol) aminated capture oligo. Following the addition of 2.5 µl of fresh 10 mg/ml 1-ethyl-3-3-dimethylaminopropyl carbodiimide (EDC), the mixture was vortexed and incubated at room temperature for 30 min in the dark. The addition of EDC and subsequent incubation was repeated. The coupled microspheres were then washed twice, first by adding 0.5 ml of 0.02% TWEEN 20^{®}, and then, after pelleting at 8,000 × g for 2 min, by resuspending in 0.5 ml of 0.1% SDS. The washed microspheres were pelleted again at 8.000 x g for 2 min, resuspended in 20 µl of Tris-EDTA (TE) buffer (pH 8.0), and stored at 4-8°C in the dark until use. For each sample batch, a fresh bead mix containing all microsphere-conjugated anti-tags, each at final concentration of 1000/µl, was prepared in 1.5x TMAC buffer.

### Hybridization with microspheres and detection

A reporter mix with fluorescent streptavidin R-phycoerythrin (SAPE) molecules attached to a common 5'-biotinylated detection probe, [Btn]Fa035(D18), was freshly prepared for each sample batch. For each 45 µl of [Btn]Fa035(D18) at 10 µM concentration, 7 µl of 1 mg/mL SAPE was added and the mixture was incubated for 60 min at 37 °C with gentle shaking. The final reporter mix consisted of SAPE-labeled detection probes diluted to 2.5 µM in 1× TMAC hybridization buffer. The actual bead array hybridizations were carried out at 55°C for 30 min in 50-µL volumes containing 5 µl of ssDNA targets, 30 µl 1.5× TMAC buffer, 13.5 µl of TE buffer (pH 8.0), and 1.5 µl bead mix. After pelleting the LUMINEX^{®} xMAP^{®} microsphere beads and removing the supernatant, the pellet was re-suspended in 40 µl SAPE-reporter mix and incubated at 55°C for 30 min. The microspheres were analyzed using a LUMINEX^{®} 200 instrument (Luminex Corporation, USA) according to the manufacturer's standard protocol. Results were recorded as the mean fluorescence intensity (MFI) associated with each type of microsphere.

### Results

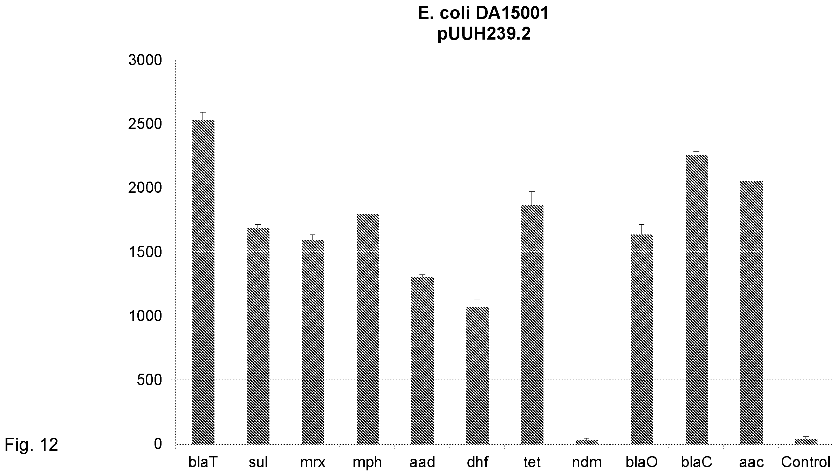

All the targeted genes were readily detected in the pUUH239.2 plasmid as displayed in Fig. 12. The measurement was done in triplicate and the positive signals, as measured in MFI, were all larger than 1000, while the negative control was below 50. LUMINEX^{®} signals are generally regarded as positive if their values are four times higher than the background.

The assessment of specificity, where one target gene product at the time was removed, demonstrated consistent results for all genes as shown in Table 4. Each measurement was done in triplicate and the MFIs of the missing genes were all on the same level as the negative controls.

**Table 4 - LUMINEX^{®} results for combined samples of all 11 genes, each with one intentionally missing**

| **Gene not present** | **blaT** | **sul** | **mrx** | **mph** | **aad** | **dhf** | **tet** | **ndm** | **blaO** | **blaC** | **aac** | **Control** |
|---|---|---|---|---|---|---|---|---|---|---|---|---|
| blaT | **44** | **875** | **1496** | **1430** | **1513** | **1110** | **1669** | **2568** | **2129** | **2861** | **2255** | **53** |
| | ±25 | ±112 | ±73 | ±80 | ±67 | ±48 | ±51 | ±71 | ±19 | ±147 | ±38 | ±7 |
| sul | **2659** | **34** | **1559** | **1473** | **1520** | **1193** | **1751** | **2609** | **2121** | **2830** | **2224** | **59** |
| | ±61 | ±11 | ±20 | ±97 | ±46 | ±15 | ±3 | ±142 | ±29 | ±30 | ±67 | ±10 |
| mrx | **2609** | **914** | **60** | **1505** | **1520** | **1182** | **1739** | **2818** | **2084** | **2690** | **2199** | **53** |
| | ±118 | ±112 | ±3 | ±164 | ±36 | ±20 | ±61 | ±81 | ±108 | ±65 | ±59 | ±10 |
| mph | **2581** | **898** | **1538** | **34** | **1532** | **1120** | **1707** | **2626** | **2050** | **2717** | **2204** | **51** |
| | ±43 | ±27 | ±36 | ±4 | ±23 | ±14 | ±15 | ±106 | ±23 | ±96 | ±58 | ±9 |
| aad | **2692** | **894** | **1610** | **1502** | **46** | **1198** | **1768** | **2731** | **2095** | **2779** | **2188** | **60** |
| | ±97 | ±88 | ±40 | ±65 | ±15 | ±3 | ±68 | ±26 | ±78 | ±92 | ±51 | ±13 |
| dhf | **2635** | **894** | **1558** | **1536** | **1538** | **37** | **1765** | **2619** | **2088** | **2771** | **2254** | **50** |
| | ±62 | ±74 | ±30 | ±104 | ±62 | ±20 | ±106 | ±3 | ±28 | ±52 | ±39 | ±9 |
| tet | **2474** | **889** | **1555** | **1475** | **1522** | **1113** | **37** | **2527** | **2032** | **2785** | **2201** | **49** |
| | ±104 | ±19 | ±41 | ±34 | ±25 | ±43 | ±12 | ±68 | ±46 | ±22 | ±32 | ±5 |
| ndm | **2643** | **866** | **1570** | **1504** | **1519** | **1182** | **1789** | **9** | **2042** | **2787** | **2215** | **63** |
| | ±36 | ±56 | ±105 | ±49 | ±65 | ±51 | ±89 | ±9 | ±58 | ±183 | ±116 | ±5 |
| blaO | **2791** | **1039** | **1624** | **1574** | **1630** | **1193** | **1915** | **2599** | **46** | **2865** | **2323** | **47** |
| | ±127 | ±107 | ±56 | ±97 | ±152 | ±61 | ±122 | ±31 | ±14 | ±86 | ±90 | ±13 |
| blaC | **2772** | **1107** | **1679** | **1694** | **1682** | **1203** | **1876** | **2653** | **2183** | **50** | **2303** | **49** |
| | ±70 | ±89 | ±54 | ±52 | ±25 | ±16 | ±42 | ±83 | ±27 | ±2 | ±39 | ±7 |
| aac | **2882** | **1029** | **1640** | **1636** | **1710** | **1253** | **1899** | **2639** | **2174** | **2933** | **50** | **60** |
| | ±71 | ±21 | ±37 | ±74 | ±27 | ±12 | ±57 | ±37 | ±24 | ±127 | ±13 | ±16 |
| All Genes | **2706** | **941** | **1618** | **1602** | **1584** | **1210** | **1846** | **2557** | **2090** | **2909** | **2258** | **52** |
| | ±37 | ±36 | ±40 | ±67 | ±44 | ±19 | ±33 | ±103 | ±22 | ±18 | ±28 | ±11 |
| NTC | **46** | **48** | **46** | **35** | **47** | **54** | **40** | **55** | **52** | **42** | **49** | **50** |
| | ±5 | ±6 | ±9 | ±14 | ±3 | ±7 | ±9 | ±4 | ±10 | ±9 | ±20 | ±11 |

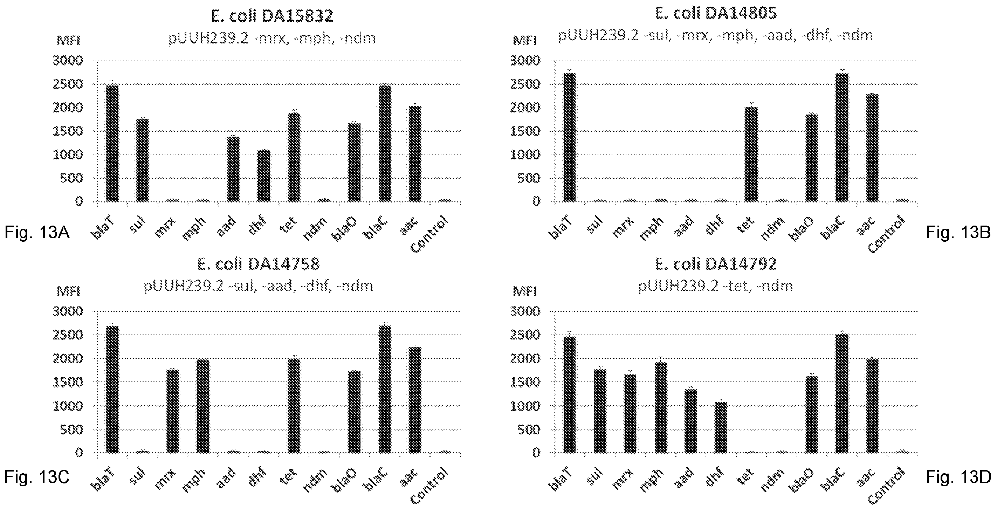

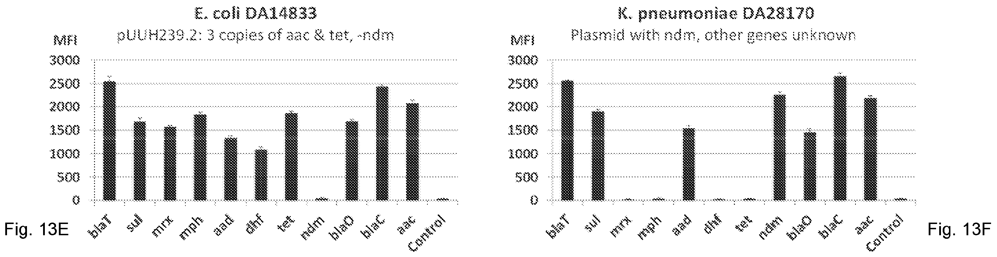

The optimised assay was used to identify resistance genes in six samples of cultivated bacteria, four containing modified versions of the pUUH239.2 plasmid and one with a largely uncharacterized plasmid carrying the NDM-1 resistance gene (as listed in Table 1). All experiments were performed in triplicate and the analysis of the bacterial samples correctly revealed their antibiotic resistance profiles and the result for each sample is presented in Figs. 13A-13F. In addition, the results indicate that the uncharacterized plasmid not only carries the NDM-1 resistance gene, but also *bla_{TEM-1}, sul1, aadA2, bla_{OXA-1}, bla_{CTX-M-15}* and *aac-(6')-1b-cr.* These findings were also verified by PCR and Sanger sequencing (data not shown).

As the results demonstrate, a novel 2-step multiplex PCR detection system has been developed and can be used for culture-independent multiplex detection of antibiotic resistance genes. Since the design allows the assay to be easily expanded or modified to fit a given set of genes, it can be readily adapted for other purposes and detection platforms.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

### REFERENCES

[1] Journal of Clinical Microbiology, 2011, 49(11): 3860-3873
[2] Archives of Virology, 2012, 157(5): 833-844
[3] WO 2011/056133
*[4]* Clinica Chimica Acta, 2006, 363: 71-82
[5] Nucleic Acids Research, 2005, 33(2): e11
*[6]* BioTechniques, 2008, 44(2): 241-248
*[7]* Journal of Clinical Microbiology, 2009, 47(2): 327-334
*[8]* Journal of Antimicrobial Chemotherapy, 2012, 67(1): 74-83

## Claims

1. A method of amplification of a target nucleic acid sequence (1), said method comprising:
a) selective amplification (S2) of said target nucleic acid sequence (1) using a set of selection primers (10, 20) and a first set of amplification primers (30, 40) in a primer-dependent enzymatic reaction to yield an amplicon (4), wherein
said set of selection primers (10, 20) comprises:
a forward selection primer (10) comprising, from a 5' end (11) to a 3' end (12), a first non-genotype specific amplification sequence (13) and a first recognition sequence (14) complementary to a first segment (2) of said target nucleic acid sequence (1); and
a reverse selection primer (20) comprising, from a 5' end (21) to a 3' end (22), a second non-genotype specific amplification sequence (23) and a second recognition sequence (24) complementary to a second segment (3) of said target nucleic acid sequence (1);
said first set of amplification primers (30, 40) comprises:
a forward amplification primer (30) corresponding or complementary to said first non-genotype specific amplification sequence (13); and
a reverse amplification primer (40) corresponding or complementary to said second non-genotype specific amplification sequence (23);
said first set of amplification primers (30, 40) is present in a concentration of at least 100:1 with regard to a concentration of said set of selection primers (10, 20); and
b) amplification and barcoding (S3) of said amplicon (4) using a barcoding primer (50) and a second set of amplification primers (30, 60; 40, 70) in a primer-dependent enzymatic reaction to yield a barcoded amplicon (6), wherein
said barcoding primer (50) comprises, from a 5' end (51) to a 3' end (52),
i) said first non-genotype specific amplification sequence (13) or a third non-genotype specific amplification sequence (53), a barcode sequence (55) and said first recognition sequence (14) or a third recognition sequence (54) complementary to a first segment (5) of said amplicon (4); or
ii) said second non-genotype specific amplification sequence (23) or a third non-genotype specific amplification sequence (53), a barcode sequence (55) and said second recognition sequence (24) or a fourth recognition sequence (56) complementary to a second segment (7) of said amplicon (4); or
iii) said barcoding primer (50) comprises a forward barcoding primer (50') and a reverse barcoding primer (50"), wherein said forward barcoding primer (50') comprises, from a 5' end (51') to a 3' end (52'), said first non-genotype specific amplification sequence (13) or a third non-genotype specific amplification sequence (53'), a first barcode sequence (55') and said first recognition sequence (14) or a third recognition sequence (54) complementary to a first segment (5) of said amplicon (4) and said reverse barcoding primer (50") comprises, from a 5' end (51") to a 3' end (52"), said second non-genotype specific amplification sequence (23) or a third non-genotype specific amplification sequence (53"), a second barcode sequence (55") and said second recognition sequence (24) or a fourth recognition sequence (56) complementary to a second segment (7) of said amplicon (4);
said second set of amplification primers (30, 60; 40, 70) comprises:
ia) a forward amplification primer (30, 60) corresponding or complementary to said first non-genotype specific amplification sequence (13) or said third non-genotype specific amplification sequence (53); and
ib) a reverse amplification primer (40) corresponding or complementary to said second non-genotype specific amplification sequence (23); or
iia) a forward amplification primer (30) corresponding or complementary to said first non-genotype specific amplification sequence (13); and
iib) a reverse amplification primer (40, 70) corresponding or complementary to said second non-genotype specific amplification sequence (23) or said third non-genotype specific amplification sequence (53); or
iiia) a forward amplification primer (30, 60) corresponding or complementary to said first non-genotype specific amplification sequence (13) or said third non-genotype specific amplification sequence (53'); and
iiib) a reverse amplification primer (40, 70) corresponding or complementary to said second non-genotype specific amplification sequence (23) or said third non-genotype specific amplification sequence (53");
said second set of amplification primers (30, 60; 40, 70) is present in a concentration of at least 100:1 with regard to a concentration of said barcoding primer (50).

2. A method of genotyping a locus (9; 9A, 9B) in a target nucleic acid sequence (1A, 1B), said locus (9; 9A, 9B) having *N*≥2 sequence variants corresponding to N genotypes, said method comprising:
a) selective amplification (S11) of said target nucleic acid sequence (1A, 1B) using N sets of selection primers (10A, 10B, 20; 10, 20A, 20B; 10A, 10B, 20A, 20B) and a first set of amplification primers (30, 40) in a primer-dependent enzymatic reaction to yield amplicons (4A, 4B), wherein
each set of selection primers (10A, 10B, 20; 10, 20A, 20B; 10A, 10B, 20A, 20B) of said N sets of selection primers (10A, 10B, 20; 10, 20A, 20B) comprises:
ia) a respective forward selection primer (10A, 10B) comprising, from a 5' end (11) to a 3' end (12), a first non-genotype specific amplification sequence (13) and a respective first locus recognition sequence (14A, 14B) complementary to a respective sequence variant of said locus (9); and
ib) a common reverse selection primer (20) comprising, from a 5' end (21) to a 3' end (22), a second non-genotype specific amplification sequence (23) and a recognition sequence (24) complementary to a second segment (3) of said target nucleic acid sequence (1A, 1B);
iia) a common forward selection primer (10) comprising, from a 5' end (11) to a 3' end (12), a first non-genotype specific amplification sequence (13) and a recognition sequence (14) complementary to a first segment (2) of said target nucleic acid sequence (1A, 1B); and
iib) a respective reverse selection primer (20A, 20B) comprising, from a 5' end (21) to a 3' end (22), a second non-genotype specific amplification sequence (23) and a respective second locus recognition sequence (24A, 24B) complementary to a respective sequence variant of said locus (9); or
iiia) a respective forward selection primer (10A, 10B) comprising, from a 5' end (11) to a 3' end (12), a first non-genotype specific amplification sequence (13) and a respective first locus recognition sequence (14A, 14B) complementary to a respective sequence variant of a first locus (9A); and
iiib) a respective reverse selection primer (20A, 20B) comprising, from a 5' end (21) to a 3' end (22), a second non-genotype specific amplification sequence (23) and a respective second locus recognition sequence (24A, 24B) complementary to a respective sequence variant of a second locus (9B);
said first set of amplification primers (30, 40) comprises:
a forward amplification primer (30) corresponding or complementary to said first non-genotype specific amplification sequence (13); and
a reverse amplification primer (40) corresponding or complementary to said second non-genotype specific amplification sequence (23);
said first set of amplification primers (30, 40) is present in a concentration of at least 100:1 with regard to a concentration of said N sets of selection primers (10A, 10B, 20; 10, 20A, 20B; 10A, 10B, 20A, 20B);
b) amplification and barcoding (S12) of said amplicons (4A, 4B) using N barcoding primers (50A, 50B) and a second set of amplification primers (30, 60; 40, 70) in a primer-dependent enzymatic reaction to yield barcoded amplicons (6A, 6B), wherein
each barcoding primer (50A, 50B) of said N barcoding primers (50A, 50B) comprises, from a 5' end (51) to a 3' end (52),
i) said first non-genotype specific amplification sequence (13) or a third non-genotype specific amplification sequence (53), a respective barcode sequence (55A, 55B) and said respective first locus recognition sequence (14A, 14B) or a respective third locus recognition sequence (54A, 54B) complementary to said respective sequence variant of said locus (9; 9A); or
ii) said second non-genotype specific amplification sequence (23) or a third non-genotype specific amplification sequence (53), a respective barcode sequence (55A, 55B) and said respective second locus recognition sequence (24A, 24B) or a respective fourth locus recognition sequence (56A, 56B) complementary to said respective sequence variant of said locus (9; 9B);
said second set of amplification primers (30, 60; 40, 70) comprises:
ia) a forward amplification primer (30, 60) corresponding or complementary to said first non-genotype specific amplification sequence (13) or said third non-genotype specific amplification sequence (53); and
ib) a reverse amplification primer (40) corresponding or complementary to said second non-genotype specific amplification sequence (23); or
iia) a forward amplification primer (30) corresponding or complementary to said first non-genotype specific amplification sequence (13); and
iib) a reverse amplification primer (40, 70) corresponding or complementary to said second non-genotype specific amplification sequence (23) or said third non-genotype specific amplification sequence (53);
said second set of amplification primers (30, 60; 40, 70) is present in a concentration of at least 100:1 with regard to a concentration of said N barcoding primers (50A, 50B); and
c) genotyping (S13) said N sequence variants of said locus (9; 9A, 9B) by:
c1a) contacting, during or after amplification and barcoding in step b), each barcoded amplicon (6A, 6B) with N labelled detection probes (90A, 90B) comprising a respective label (92A, 92B) and a respective sequence (91A, 91B) complementary to a barcode sequence (55A, 55B) of said N barcode sequences (55A, 55B); and
c1b) relating an amount of detected label (92A, 92B) to a specific genotype for said locus (9; 9A, 9B); or
c2a) contacting, after amplification and barcoding in step b), each barcoded amplicon (6A, 6B) with a detection array of N genotype specific sequences, each genotype specific sequence of said N genotype specific sequences is complementary to a respective barcode sequence (55A, 55B) of said N barcode sequences (55A, 55B); and
c2b) relating detected hybridization of each barcoded amplicon (6A, 6B) to said detection array to a specific genotype for said locus (9; 9A, 9B).

3. The method according to claim 2, wherein
contacting each barcoded amplicon in step c1a) comprises contacting, during or after amplification and barcoding in step b), each barcoded amplicon (6A, 6B) with a labelled common probe complementary to said first non-genotype specific amplification sequence (13) or said third non-genotype specific amplification sequence (53) and N labelled detection probes comprising a respective labelled bead and said respective sequence complementary to a barcode sequence (55A, 55B) of said N barcode sequences (55A, 55B); and
relating said amount of detected label in step c1b) comprises relating an amount of detected labelled bead to said specific genotype for said locus (9; 9A, 9B).

4. The method according to any of the claims 1 to 3, further comprising pre-amplification (S1, S10) of said target nucleic acid sequence (1; 1A, 1B) in a primer-dependent enzymatic reaction.

5. The method according to claim 4, wherein pre-amplification (S1, S10) of said target nucleic acid sequence (1; 1A, 1B) comprises:
multiple displacement amplification of said target nucleic acid sequence (1; 1A, 1B) in a primer-dependent enzymatic reaction; or
sequence-independent amplification of said target nucleic acid sequence (1; 1A, 1B) in a primer-dependent enzymatic reaction.

6. The method according to any of the claims 1 to 5, wherein a 5' end of said forward amplification primer (60) of said second set of amplification primers (30, 60; 40, 70) comprises a phosphate group, said method further comprises:
generating (S20) single-stranded barcoded amplicons (6A, 6B) by treating said barcoded amplicons (6A, 6B) after step b) with an exonuclease.

7. A kit for amplification of a target nucleic acid sequence (1), said kit comprising:
a set of selection primers (10, 20) comprising:
a forward selection primer (10) comprising, from a 5' end (11) to a 3' end (12), a first non-genotype specific amplification sequence (13) and a first recognition sequence (14) complementary to a first segment (2) of said target nucleic acid sequence (1); and
a reverse selection primer (20) comprising, from a 5' end (21) to a 3' end (22), a second non-genotype specific amplification sequence (23) and a second recognition sequence (24) complementary to a second segment (3) of said target nucleic acid sequence (1);
a first set of amplification primers (30, 40) comprising:
a forward amplification primer (30) corresponding or complementary to said first non-genotype specific amplification sequence (13); and
a reverse amplification primer (40) corresponding or complementary to said second non-genotype specific amplification sequence (23);
said forward selection primer (10) and said reverse selection primer (20) are present in a concentration of less than 100 nM;
said first set of amplification primers (30, 40) is present in a concentration of at least 100:1 with regard to a concentration of said set of selection primers (10, 20); and
a barcoding primer (50) comprising, from a 5' end (51) to a 3' end (52),
i) said first non-genotype specific amplification sequence (13) or a third non-genotype specific amplification sequence (53), a barcode sequence (55) and said first recognition sequence (14) or a third recognition sequence (54) complementary to a first segment (5) of said amplicon (4); or
ii) said second non-genotype specific amplification sequence (23) or a third non-genotype specific amplification sequence (53), a barcode sequence (55) and said second recognition sequence (24) or a fourth recognition sequence (56) complementary to a second segment (7) of said amplicon (4); or
iii) said barcoding primer (50) comprises a forward barcoding primer (50') and a reverse barcoding primer (50"), wherein said forward barcoding primer (50') comprises, from a 5' end (51') to a 3' end (52'), said first non-genotype specific amplification sequence (13) or a third non-genotype specific amplification sequence (53'), a first barcode sequence (55') and said first recognition sequence (14) or a third recognition sequence (54) complementary to a first segment (5) of said amplicon (4) and said reverse barcoding primer (50") comprises, from a 5' end (51") to a 3' end (52"), said second non-genotype specific amplification sequence (23) or a third non-genotype specific amplification sequence (53"), a second barcode sequence (55") and said second recognition sequence (24) or a fourth recognition sequence (56) complementary to a second segment (7) of said amplicon (4);
a second set of amplification primers (30, 60; 40, 70) comprising:
ia) a forward amplification primer (30, 60) corresponding or complementary to said first non-genotype specific amplification sequence (13) or said third non-genotype specific amplification sequence (53); and
ib) a reverse amplification primer (40) corresponding or complementary to said second non-genotype specific amplification sequence (23); or
iia) a forward amplification primer (30) corresponding or complementary to said first non-genotype specific amplification sequence (13); and
iib) a reverse amplification primer (40, 70) corresponding or complementary to said second non-genotype specific amplification sequence (23) or said third non-genotype specific amplification sequence (53); or
iiia) a forward amplification primer (30, 60) corresponding or complementary to said first non-genotype specific amplification sequence (13) or said third non-genotype specific amplification sequence (53'); and
iiib) a reverse amplification primer (40, 70) corresponding or complementary to said second non-genotype specific amplification sequence (23) or said third non-genotype specific amplification sequence (53");
said barcoding primer (50) is present in a concentration of less than 100 nM; and
said second set of amplification primers (30, 60; 40, 70) is present in a concentration of at least 100:1 with regard to a concentration of said barcoding primer (50).

8. The kit according to claim 7, wherein
said barcoding primer (50) comprises, from said 5' end (51) to said 3' end (52), said first non-genotype specific amplification sequence (13) or said third non-genotype specific amplification sequence (53), preferably said third non-genotype specific amplification sequence (53), said barcode sequence (55) and said first recognition sequence (14) or said third recognition sequence (54); and
said second set of amplification primers (30, 60; 40, 70) comprises:
said forward amplification primer (30, 60) corresponding or complementary to said first non-genotype specific amplification sequence (13) or said third non-genotype specific amplification sequence (53), preferably said third non-genotype specific amplification sequence (53); and
said reverse amplification primer (40) corresponding or complementary to said second non-genotype specific amplification sequence (23).

9. The kit according to claim 7 or 8, wherein
said forward selection primer (10) and said reverse selection primer (20) are present in a concentration of from 0.01 to 10 nM; and
said barcoding primer (50) is present in a concentration of from 0.01 to 10 nM.

10. The kit according to any of the claims 7 to 9, wherein
said first set of amplification primers (30, 40) is present in a concentration of from 100:1 to 100,000:1 with regard to said concentration of said set of selection primers (10, 20); and
said second set of amplification primers (30, 60; 40, 70) is present in a concentration of from 100:1 to 100,000:1 with regard to said concentration of said barcoding primer (50).

11. A kit for genotyping a locus (9; 9A, 9B) in a target nucleic acid sequence (1A, 1B), said locus (9; 9A, 9B) having *N*≥2 sequence variants corresponding to N genotypes, said kit comprising:
N sets of selection primers (10A, 10B, 20; 10, 20A, 20B; 10A, 10B, 20A, 20B), wherein each set of selection primers (10A, 10B, 20; 10, 20A, 20B; 10A, 10B, 20A, 20B) of said N sets of selection primers (10A, 10B, 20; 10, 20A, 20B; 10A, 10B, 20A, 20B) comprises:
ia) a respective forward selection primer (10A, 10B) comprising, from a 5' end (11) to a 3' end (12), a first non-genotype specific amplification sequence (13) and a respective first locus recognition sequence (14A, 14B) complementary to a respective sequence variant of said locus (9); and
ib) a common reverse selection primer (20) comprising, from a 5' end (21) to a 3' end (22), a second non-genotype specific amplification sequence (23) and a recognition sequence (24) complementary to a second segment (3) of said target nucleic acid sequence (1);
iia) a common forward selection primer (10) comprising, from a 5' end (11) to a 3' end (12), a first non-genotype specific amplification sequence (13) and a recognition sequence (14) complementary to a first segment (2) of said target nucleic acid sequence (1); and
iib) a respective reverse selection primer (20A, 20B) comprising, from a 5' end (21) to a 3' end (22), a second non-genotype specific amplification sequence (23) and a respective second locus recognition sequence (24A, 24B) complementary to a respective sequence variant of said locus (9); or
iiia) a respective forward selection primer (10A, 10B) comprising, from a 5' end (11) to a 3' end (12), a first non-genotype specific amplification sequence (13) and a respective first locus recognition sequence (14A, 14B) complementary to a respective sequence variant of a first locus (9A); and
iiib) a respective reverse selection primer (20A, 20B) comprising, from a 5' end (21) to a 3' end (22), a second non-genotype specific amplification sequence (23) and a respective second locus recognition sequence (24A, 24B) complementary to a respective sequence variant of a second locus (9B);
a first set of amplification primers (30, 40) comprising:
a forward amplification primer (30) corresponding or complementary to said first non-genotype specific amplification sequence (13); and
a reverse amplification primer (40) corresponding or complementary to said second non-genotype specific amplification sequence (23);
i) each respective forward selection primer (10A, 10B) and said common reverse selection primer (20);
ii) said common forward selection primer (10) and each respective reverse selection primer (20A, 20B); or
iii) each respective forward selection primer (10A, 10B) and each respective reverse selection primer (20A, 20B), is present in a concentration of less than 100 nM;
said first set of amplification primers (30, 40) is present in a concentration of at least 100:1 with regard to a concentration of said *N* sets of selection primers (10A, 10B, 20; 10, 20A, 20B; 10A, 10B, 20A, 20B);
*N* barcoding primers (50A, 50B), wherein each barcoding primer (50A, 50B) of said *N* barcoding primers (50A, 50B) comprises, from a 5' end (51) to a 3' end (52),
i) said first non-genotype specific amplification sequence (13) or a third non-genotype specific amplification sequence (53), a respective barcode sequence (55A, 55B) and said respective first locus recognition sequence (14A, 14B) or a respective third locus recognition sequence (54A, 54B) complementary to said respective sequence variant of said locus (9); or
ii) said second non-genotype specific amplification sequence (23) or a third non-genotype specific amplification sequence (53), a respective barcode sequence (55A, 55B) and said respective second locus recognition sequence (24A, 24B) or a respective fourth locus recognition sequence (56A, 56B) complementary to said respective sequence variant of said locus (9);
a second set of amplification primers (30, 60; 40, 70) comprises:
ia) a forward amplification primer (30, 60) corresponding or complementary to said first non-genotype specific amplification sequence (13) or said third non-genotype specific amplification sequence (53); and
ib) a reverse amplification primer (40) corresponding or complementary to said second non-genotype specific amplification sequence (23); or
iia) a forward amplification primer (30) corresponding or complementary to said first non-genotype specific amplification sequence (13); and
iib) a reverse amplification primer (40, 70) corresponding or complementary to said second non-genotype specific amplification sequence (23) or said third non-genotype specific amplification sequence (53);
each barcoding primer (50A, 50B) is present in a concentration of less than 100 nM;
said second set of amplification primers (30, 60; 40, 70) is present in a concentration of at least 100:1 with regard to a concentration of said *N* barcoding primers (50A, 50B); and
*N* labelled detection probes (90A, 90B) comprising a respective label (92A, 92B) and a respective sequence (91A, 91B) complementary to a barcode sequence (55A, 55B) of said *N* barcode sequences (55A, 55B); or
a detection array of *N* genotype specific sequences, each genotype specific sequence of said *N* genotype specific sequences is complementary to a respective barcode sequence (55A, 55B) of said *N* barcode sequences (55A, 55B).

12. The kit according to claim 11, wherein
said *N* labelled detection probes comprises a respective labelled bead and said respective sequence complementary to a barcode sequence (55A, 55B) of said *N* barcode sequences (55A, 55B), said kit further comprising:
a labelled common probe complementary to said first non-genotype specific amplification sequence (13) or said third non-genotype specific amplification sequence (53).

13. The kit according to claim 11 or 12, wherein
i) each respective forward selection primer (10A, 10B) and said common reverse selection primer (20);
ii) said common forward selection primer (10) and each respective reverse selection primer (20A, 20B); or
iii) each respective forward selection primer (10A, 10B) and each respective reverse selection primer (20A, 20B), is present in a concentration of from 0.01 to 10 nM; and
each barcoding primer (50A, 50B) is present in a concentration of from 0.01 to 10 nM.

14. The kit according to any of the claims 11 to 13, wherein
said first set of amplification primers (30, 40) is present in a concentration of from 100:1 to 100,000:1 with regard to said concentration of said *N* sets of selection primers (10A, 10B, 20; 10, 20A, 20B; 10A, 10B, 20A, 20B); and
said second set of amplification primers (30, 60; 40, 70) is present in a concentration of from 100:1 to 100,000:1 with regard to said concentration of said *N* barcoding primers (50A, 50B).

15. The kit according to any of the claims 7 to 14, wherein a 5' end of said forward amplification primer (30, 60) of said second set of amplification primers (30, 60; 40, 70) comprises a phosphate group.

## Patentansprüche

1. Verfahren zur Amplifikation einer Zielnukleinsäuresequenz (1), wobei das Verfahren Folgendes umfasst:
a) eine selektive Amplifikation (S2) der Zielnukleinsäuresequenz (1) unter Verwendung eines Satzes von Selektionsprimern (10, 20) und eines ersten Satzes von Amplifikationsprimern (30, 40) in einer primerabhängigen enzymatischen Reaktion, um ein Amplicon (4) zu erhalten, wobei der Satz von Selektionsprimern (10, 20) Folgendes umfasst:
einen Vorwärtsselektionsprimer (10), der von einem 5'-Ende (11) zu einem 3'-Ende (12) eine erste nicht-genotypspezifische Amplifikationssequenz (13) und eine erste Erkennungssequenz (14) umfasst, die komplementär zu einem ersten Segment (2) der Zielnukleinsäuresequenz (1) sind; und
einen Rückwärtsselektionsprimer (20), der von einem 5'-Ende (21) zu einem 3'-Ende (22) eine zweite nicht-genotypspezifische Amplifikationssequenz (23) und eine zweite Erkennungssequenz (24) umfasst, die komplementär zu einem zweiten Segment (3) der Zielnukleinsäuresequenz (1) sind;
wobei der erste Satz von Amplifikationsprimern (30, 40) Folgendes umfasst:
einen Vorwärtsamplifikationsprimer (30), welcher der ersten nicht-genotypspezifischen Amplifikationssequenz (13) entspricht oder komplementär zu dieser ist; und
einen Rückwärtsamplifikationsprimer (40), welcher der zweiten nicht-genotypspezifischen Amplifikationssequenz (23) entspricht oder komplementär zu dieser ist;
wobei der erste Satz von Amplifikationsprimern (30, 40) in einer Konzentration von mindestens 100:1 hinsichtlich einer Konzentration des Satzes von Selektionsprimern (10, 20) vorhanden ist; und
b) eine Amplifikation und ein Barcoding (S3) des Amplicons (4) unter Verwendung eines Barcoding-Primers (50) und eines zweiten Satzes von Amplifikationsprimern (30, 60; 40, 70) in einer primerabhängigen enzymatischen Reaktion, um ein barcodiertes Amplicon (6) zu ergeben, wobei
der Barcoding-Primer (50) von einem 5'-Ende (51) zu einem 3'-Ende (52) Folgendes umfasst:
i) die erste nicht-genotypspezifische Amplifikationssequenz (13) oder eine dritte nicht-genotypspezifische Amplifikationssequenz (53), eine Barcode-Sequenz (55) und die erste Erkennungssequenz (14) oder eine dritte Erkennungssequenz (54), die komplementär zu einem ersten Segment (5) des Amplicons (4) ist/sind; oder
ii) die zweite nicht-genotypspezifische Amplifikationssequenz (23) oder eine dritte nicht-genotypspezifische Amplifikationssequenz (53), eine Barcode-Sequenz (55) und die zweite Erkennungssequenz (24) oder eine vierte Erkennungssequenz (56), die komplementär zu einem zweiten Segment (7) des Amplicons (4) ist/sind; oder
iii) der Barcoding-Primer (50) einen Vorwärtsbarcoding-Primer (50') und einen Rückwärtsbarcoding-Primer (50") umfasst, wobei der Vorwärtsbarcoding-Primer (50') von einem 5'-Ende (51') zu einem 3'-Ende (52') die erste nicht-genotypspezifische Amplifikationssequenz (13) oder eine dritte nicht-genotypspezifische Amplifikationssequenz (53'), eine erste Barcode-Sequenz (55') und die erste Erkennungssequenz (14) oder eine dritte Erkennungssequenz (54) umfasst, die komplementär zu einem ersten Segment (5) des Amplicons (4) ist/sind, und wobei der Rückwärtsbarcoding-Primer (50") von einem 5'-Ende (51") zu einem 3'-Ende (52") die zweite nicht-genotypspezifische Amplifikationssequenz (23) oder eine dritte nicht-genotypspezifische Amplifikationssequenz (53"), eine zweite Barcode-Sequenz (55") und die zweite Erkennungssequenz (24) oder eine vierte Erkennungssequenz (56) umfasst, die komplementär zu einem zweiten Segment (7) des Amplicons (4) ist/sind;
wobei der zweite Satz von Amplifikationsprimern (30, 60; 40, 70) Folgendes umfasst:
ia) einen Vorwärtsamplifikationsprimer (30, 60), welcher der ersten nicht-genotypspezifischen Amplifikationssequenz (13) oder der dritten nicht-genotypspezifischen Amplifikationssequenz (53) entspricht oder komplementär zu dieser ist; und
ib) einen Rückwärtsamplifikationsprimer (40), welcher der zweiten nicht-genotypspezifischen Amplifikationssequenz (23) entspricht oder komplementär zu dieser ist; oder
iia) einen Vorwärtsamplifikationsprimer (30), welcher der ersten nicht-genotypspezifischen Amplifikationssequenz (13) entspricht oder komplementär zu dieser ist; und
iib) einen Rückwärtsamplifikationsprimer (40, 70), welcher der zweiten nicht-genotypspezifischen Amplifikationssequenz (23) oder der dritten nicht-genotypspezifischen Amplifikationssequenz (53) entspricht oder komplementär zu dieser ist; oder
iiia) einen Vorwärtsamplifikationsprimer (30, 60), welcher der ersten nicht-genotypspezifischen Amplifikationssequenz (13) oder der dritten nicht-genotypspezifischen Amplifikationssequenz (53') entspricht oder komplementär zu dieser ist; und
iiib) einen Rückwärtsamplifikationsprimer (40, 70), welcher der zweiten nicht-genotypspezifischen Amplifikationssequenz (23) oder der dritten nicht-genotypspezifischen Amplifikationssequenz (53") entspricht oder komplementär zu dieser ist;
wobei der zweite Satz von Amplifikationsprimern (30, 60; 40, 70) in einer Konzentration von mindestens 100:1 hinsichtlich einer Konzentration des Barcoding-Primers (50) vorhanden ist.

2. Verfahren zur Genotypisierung eines Lokus (9; 9A, 9B) in einer Zielnukleinsäuresequenz (1A, 1B), wobei der Lokus (9; 9A, 9B) N≥2 Sequenzvarianten aufweist, die N Genotypen entsprechen, wobei das Verfahren Folgendes umfasst:
a) eine selektive Amplifikation (S11) der Zielnukleinsäuresequenz (1A, 1B) unter Verwendung von N Sätzen von Selektionsprimern (10A, 10B, 20; 10, 20A, 20B; 10A, 10B, 20A, 20B) und einem ersten Satz von Amplifikationsprimern (30, 40) in einer primerabhängigen enzymatischen Reaktion, um Amplicons (4A, 4B) zu erhalten, wobei
jeder Satz von Selektionsprimern (10A, 10B, 20; 10, 20A, 20B; 10A, 10B, 20A, 20B) der N Sätze von Selektionsprimern (10A, 10B, 20; 10, 20A, 20B) Folgendes umfasst:
ia) einen jeweiligen Vorwärtsselektionsprimer (10A, 10B), der von einem 5'-Ende (11) zu einem 3'-Ende (12) eine erste nicht-genotypspezifische Amplifikationssequenz (13) und eine jeweilige erste Lokuserkennungssequenz (14A, 14B) umfasst, die komplementär zu einer jeweiligen Sequenzvariante des Lokus (9) sind; und
ib) einen üblichen Rückwärtsselektionsprimer (20), der von einem 5'-Ende (21) zu einem 3'-Ende (22) eine zweite nicht-genotypspezifische Amplifikationssequenz (23) und eine Erkennungssequenz (24) umfasst, die komplementär zu einem zweiten Segment (3) der Zielnukleinsäuresequenz (1A, 1B) sind;
iia) einen üblichen Vorwärtsselektionsprimer (10), der von einem 5'-Ende (11) zu einem 3'-Ende (12) eine erste nicht-genotypspezifische Amplifikationssequenz (13) und eine Erkennungssequenz (14) umfasst, die komplementär zu einem ersten Segment (2) der Zielnukleinsäuresequenz (1A, 1B) sind; und
iib) einen jeweiligen Rückwärtsselektionsprimer (20A, 20B), der von einem 5'-Ende (21) zu einem 3'-Ende (22) eine zweite nicht-genotypspezifische Amplifikationssequenz (23) und eine jeweilige zweite Lokuserkennungssequenz (24A, 24B) umfasst, die komplementär zu einer jeweiligen Sequenzvariante des Lokus (9) sind; oder
iiia) einen jeweiligen Vorwärtsselektionsprimer (10A, 10B), der von einem 5'-Ende (11) zu einem 3'-Ende (12) eine erste nicht-genotypspezifische Amplifikationssequenz (13) und eine jeweilige erste Lokuserkennungssequenz (14A, 14B) umfasst, die komplementär zu einer jeweiligen Sequenzvariante eines ersten Lokus (9A) sind; und
iiib) einen jeweiligen Rückwärtsselektionsprimer (20A, 20B), der von einem 5'-Ende (21) zu einem 3'-Ende (22) eine zweite nicht-genotypspezifische Amplifikationssequenz (23) und eine jeweilige zweite Lokuserkennungssequenz (24A, 24B) umfasst, die komplementär zu einer jeweiligen Sequenzvariante eines zweiten Lokus (9B) sind;
wobei der erste Satz von Amplifikationsprimern (30, 40) Folgendes umfasst:
einen Vorwärtsamplifikationsprimer (30), welcher der ersten nicht-genotypspezifischen Amplifikationssequenz (13) entspricht oder komplementär zu dieser ist; und
einen Rückwärtsamplifikationsprimer (40), welcher der zweiten nicht-genotypspezifischen Amplifikationssequenz (23) entspricht oder komplementär zu dieser ist;
wobei der erste Satz von Amplifikationsprimern (30, 40) in einer Konzentration von mindestens 100:1 hinsichtlich einer Konzentration der N Sätze von Selektionsprimern (10A, 10B, 20; 10, 20A, 20B; 10A, 10B, 20A, 20B) vorhanden ist;
b) eine Amplifikation und ein Barcoding (S12) der Amplicons (4A, 4B) unter Verwendung von N Barcoding-Primern (50A, 50B) und einem zweiten Satz von Amplifikationsprimern (30, 60; 40, 70) in einer primerabhängigen enzymatischen Reaktion, um barcodierte Amplicons (6A, 6B) zu ergeben, wobei
jeder Barcoding-Primer (50A, 50B) der N Barcoding-Primer (50A, 50B) von einem 5'-Ende (51) zu einem 3'-Ende (52) Folgendes umfasst:
i) die erste nicht-genotypspezifische Amplifikationssequenz (13) oder eine dritte nicht-genotypspezifische Amplifikationssequenz (53), eine jeweilige Barcode-Sequenz (55A, 55B) und die jeweilige erste Lokuserkennungssequenz (14A, 14B) oder eine jeweilige dritte Lokuserkennungssequenz (54A, 54B), die komplementär zu der jeweiligen Sequenzvariante des Lokus (9; 9A) ist/sind; oder
ii) die zweite nicht-genotypspezifische Amplifikationssequenz (23) oder eine dritte nicht-genotypspezifische Amplifikationssequenz (53), eine jeweilige Barcode-Sequenz (55A, 55B) und die jeweilige zweite Lokuserkennungssequenz (24A, 24B) oder eine jeweilige vierte Lokuserkennungssequenz (56A, 56B), die komplementär zu der jeweiligen Sequenzvariante des Lokus (9; 9B) ist/sind;
wobei der zweite Satz von Amplifikationsprimern (30, 60; 40, 70) Folgendes umfasst:
ia) einen Vorwärtsamplifikationsprimer (30, 60), welcher der ersten nicht-genotypspezifischen Amplifikationssequenz (13) oder der dritten nicht-genotypspezifischen Amplifikationssequenz (53) entspricht oder komplementär zu dieser ist; und
ib) einen Rückwärtsamplifikationsprimer (40), welcher der zweiten nicht-genotypspezifischen Amplifikationssequenz (23) entspricht oder komplementär zu dieser ist; oder
iia) einen Vorwärtsamplifikationsprimer (30), welcher der ersten nicht-genotypspezifischen Amplifikationssequenz (13) entspricht oder komplementär zu dieser ist; und
iib) einen Rückwärtsamplifikationsprimer (40, 70), welcher der zweiten nicht-genotypspezifischen Amplifikationssequenz (23) oder der dritten nicht-genotypspezifischen Amplifikationssequenz (53) entspricht oder komplementär zu dieser ist;
wobei der zweite Satz von Amplifikationsprimern (30, 60; 40, 70) in einer Konzentration von mindestens 100:1 hinsichtlich einer Konzentration der *N* Barcoding-Primer (50A, 50B) vorhanden ist; und
c) eine Genotypisierung (S13) der *N* Sequenzvarianten des Lokus (9; 9A, 9B) durch Folgendes:
c1a) Inkontaktbringen jedes barcodierten Amplicons (6A, 6B), während der Amplifikation und des Barcoding in Schritt b) oder danach, mit *N* markierten Detektionssonden (90A, 90B), die eine jeweilige Markierung (92A, 92B) und eine jeweilige Sequenz (91A, 91B) umfassen, die komplementär zu einer Barcode-Sequenz (55A, 55B) der *N* Barcode-Sequenzen (55A, 55B) ist; und
c1b) Inbezugbringen eines Anteils der detektierten Markierung (92A, 92B) mit einem spezifischen Genotyp des Lokus (9; 9A, 9B); oder
c2a) Inkontaktbringen jedes barcodierten Amplicons (6A, 6B), nach der Amplifikation und dem Barcoding in Schritt b), mit einer Detektionsanordnung von *N* genotypspezifischen Sequenzen, wobei jede genotypspezifische Sequenz der *N* genotypspezifischen Sequenzen komplementär zu einer jeweiligen Barcode-Sequenz (55A, 55B) der *N* Barcode-Sequenzen (55A, 55B) ist; und
c2b) Inbezugbringen einer detektierten Hybridisierung jedes barcodierten Amplicons (6A, 6B) an der Detektionsanordnung mit einem spezifischen Genotyp für den Lokus (9; 9A, 9B).

3. Verfahren nach Anspruch 2, wobei
das Inkontaktbringen jedes barcodierten Amplicons in Schritt c1a) Folgendes umfasst: Inkontaktbringen jedes barcodierten Amplicons (6A, 6B), während der Amplifikation und des Barcoding in Schritt b) oder danach, mit einer markierten üblichen Sonde, die komplementär zu der ersten nicht-genotypspezifischen Amplifikationssequenz (13) oder der dritten nicht-genotypspezifischen Amplifikationssequenz (53) ist, oder *N* markierten Detektionssonden, die ein jeweiliges markiertes Kügelchen und die jeweilige Sequenz umfasst, die komplementär zu einer Barcode-Sequenz (55A, 55B) der *N* Barcode-Sequenzen (55A, 55B) ist; und
das Inbezugbringen des Anteils der detektierten Markierung bei Schritt c1b) Inbezugbringen eines Anteils eines detektierten markierten Kügelchens mit dem spezifischen Genotyp für den Lokus (9; 9A, 9B) umfasst.

4. Verfahren nach einem der Ansprüche 1 bis 3, ferner umfassend eine Voramplifikation (S1, S10) der Zielnukleinsäuresequenz (1; 1A, 1B) in einer primerabhängigen enzymatischen Reaktion.

5. Verfahren nach Anspruch 4, wobei die Voramplifikation (S1, S10) der Zielnukleinsäuresequenz (1; 1A, 1B) Folgendes umfasst:
eine Multiple Displacement Amplification der Zielnukleinsäuresequenz (1; 1A, 1B) in einer primerabhängigen enzymatischen Reaktion; oder
eine sequenzunabhängige Amplifikation der Zielnukleinsäuresequenz (1; 1A, 1B) in einer primerabhängigen enzymatischen Reaktion.

6. Verfahren nach einem der Ansprüche 1 bis 5, wobei ein 5'-Ende des Vorwärtsamplifikationsprimers (60) des zweiten Satzes von Amplifikationsprimern (30, 60; 40, 70) eine Phosphatgruppe umfasst, wobei das Verfahren ferner Folgendes umfasst:
Erzeugen (S20) von einsträngigen barcodierten Amplicons (6A, 6B) durch Behandeln der barcodierten Amplicons (6A, 6B) nach Schritt b) mit einer Exonuklease.

7. Kit zur Amplifikation einer Zielnukleinsäuresequenz (1), wobei das Kit Folgendes umfasst:
einen Satz von Selektionsprimern (10, 20), der Folgendes umfasst:
einen Vorwärtsselektionsprimer (10), der von einem 5'-Ende (11) zu einem 3'-Ende (12) eine erste nicht-genotypspezifische Amplifikationssequenz (13) und eine erste Erkennungssequenz (14) umfasst, die komplementär zu einem ersten Segment (2) der Zielnukleinsäuresequenz (1) sind; und
einen Rückwärtsselektionsprimer (20), der von einem 5'-Ende (21) zu einem 3'-Ende (22) eine zweite nicht-genotypspezifische Amplifikationssequenz (23) und eine zweite Erkennungssequenz (24) umfasst, die komplementär zu einem zweiten Segment (3) der Zielnukleinsäuresequenz (1) sind;
einen ersten Satz von Amplifikationsprimern (30, 40), der Folgendes umfasst:
einen Vorwärtsamplifikationsprimer (30), welcher der ersten nicht-genotypspezifischen Amplifikationssequenz (13) entspricht oder komplementär zu dieser ist; und
einen Rückwärtsamplifikationsprimer (40), welcher der zweiten nicht-genotypspezifischen Amplifikationssequenz (23) entspricht oder komplementär zu dieser ist;
wobei der Vorwärtsselektionsprimer (10) und der Rückwärtsselektionsprimer (20) in einer Konzentration von weniger als 100 nM vorhanden sind;
wobei der erste Satz von Amplifikationsprimern (30, 40) in einer Konzentration von mindestens 100:1 hinsichtlich einer Konzentration des Satzes von Selektionsprimern (10, 20) vorhanden ist; und
einen Barcoding-Primer (50), der von einem 5'-Ende (51) zu einem 3'-Ende (52) Folgendes umfasst:
i) die erste nicht-genotypspezifische Amplifikationssequenz (13) oder eine dritte nicht-genotypspezifische Amplifikationssequenz (53), eine Barcode-Sequenz (55) und die erste Erkennungssequenz (14) oder eine dritte Erkennungssequenz (54), die komplementär zu einem ersten Segment (5) des Amplicons (4) ist/sind; oder
ii) die zweite nicht-genotypspezifische Amplifikationssequenz (23) oder eine dritte nicht-genotypspezifische Amplifikationssequenz (53), eine Barcode-Sequenz (55) und die zweite Erkennungssequenz (24) oder eine vierte Erkennungssequenz (56), die komplementär zu einem zweiten Segment (7) des Amplicons (4) ist/sind; oder
iii) der Barcoding-Primer (50) einen Vorwärtsbarcoding-Primer (50') und einen Rückwärtsbarcoding-Primer (50") umfasst, wobei der Vorwärtsbarcoding-Primer (50') von einem 5'-Ende (51') zu einem 3'-Ende (52') die erste nicht-genotypspezifische Amplifikationssequenz (13) oder eine dritte nicht-genotypspezifische Amplifikationssequenz (53'), eine erste Barcode-Sequenz (55') und die erste Erkennungssequenz (14) oder eine dritte Erkennungssequenz (54) umfasst, die komplementär zu einem ersten Segment (5) des Amplicons (4) ist/sind, und wobei der Rückwärtsbarcoding-Primer (50") von einem 5'-Ende (51") zu einem 3'-Ende (52") die zweite nicht-genotypspezifische Amplifikationssequenz (23) oder eine dritte nicht-genotypspezifische Amplifikationssequenz (53"), eine zweite Barcode-Sequenz (55") und die zweite Erkennungssequenz (24) oder eine vierte Erkennungssequenz (56) umfasst, die komplementär zu einem zweiten Segment (7) des Amplicons (4) ist/sind;
wobei ein zweiter Satz von Amplifikationsprimern (30, 60; 40, 70) Folgendes umfasst:
ia) einen Vorwärtsamplifikationsprimer (30, 60), welcher der ersten nicht-genotypspezifischen Amplifikationssequenz (13) oder der dritten nicht-genotypspezifischen Amplifikationssequenz (53) entspricht oder komplementär zu dieser ist; und
ib) einen Rückwärtsamplifikationsprimer (40), welcher der zweiten nicht-genotypspezifischen Amplifikationssequenz (23) entspricht oder komplementär zu dieser ist; oder
iia) einen Vorwärtsamplifikationsprimer (30), welcher der ersten nicht-genotypspezifischen Amplifikationssequenz (13) entspricht oder komplementär zu dieser ist; und
iib) einen Rückwärtsamplifikationsprimer (40, 70), welcher der zweiten nicht-genotypspezifischen Amplifikationssequenz (23) oder der dritten nicht-genotypspezifischen Amplifikationssequenz (53) entspricht oder komplementär zu dieser ist; oder
iiia) einen Vorwärtsamplifikationsprimer (30, 60), welcher der ersten nicht-genotypspezifischen Amplifikationssequenz (13) oder der dritten nicht-genotypspezifischen Amplifikationssequenz (53') entspricht oder komplementär zu dieser ist; und
iiib) einen Rückwärtsamplifikationsprimer (40, 70), welcher der zweiten nicht-genotypspezifischen Amplifikationssequenz (23) oder der dritten nicht-genotypspezifischen Amplifikationssequenz (53") entspricht oder komplementär zu dieser ist;
wobei der Barcoding-Primer (50) in einer Konzentration von weniger als 100 nM vorhanden ist; und
wobei der zweite Satz von Amplifikationsprimern (30, 60; 40, 70) in einer Konzentration von mindestens 100:1 hinsichtlich einer Konzentration des Barcoding-Primers (50) vorhanden ist.

8. Kit nach Anspruch 7, wobei
der Barcoding-Primer (50) von dem 5'-Ende (51) zu dem 3'-Ende (52) die erste nicht-genotypspezifische Amplifikationssequenz (13) oder die dritte nicht-genotypspezifische Amplifikationssequenz (53), vorzugsweise die dritte nicht-genotypspezifische Amplifikationssequenz (53), die Barcode-Sequenz (55) und die erste Erkennungssequenz (14) oder die dritte Erkennungssequenz (54) umfasst; und
der zweite Satz von Amplifikationsprimern (30, 60; 40, 70) Folgendes umfasst:
den Vorwärtsamplifikationsprimer (30, 60), welcher der ersten nicht-genotypspezifischen Amplifikationssequenz (13) oder der dritten nicht-genotypspezifischen Amplifikationssequenz (53'), vorzugsweise der dritten nicht-genotypspezifischen Amplifikationssequenz (53), entspricht oder komplementär zu dieser ist; und
den Rückwärtsamplifikationsprimer (40), welcher der zweiten nicht-genotypspezifischen Amplifikationssequenz (23) entspricht oder komplementär zu dieser ist.

9. Kit nach Anspruch 7 oder 8, wobei
der Vorwärtsselektionsprimer (10) und der Rückwärtsselektionsprimer (20) in einer Konzentration von 0,01 bis 10 nM vorhanden sind; und
der Barcoding-Primer (50) in einer Konzentration von 0,01 bis 10 nM vorhanden ist.

10. Kit nach einem der Ansprüche 7 bis 9, wobei der erste Satz von Amplifikationsprimern (30, 40) in einer Konzentration von 100:1 bis 100.000:1 hinsichtlich der Konzentration des Satzes von Selektionsprimern (10, 20) vorhanden ist; und
der zweite Satz von Amplifikationsprimern (30, 60; 40, 70) in einer Konzentration von 100:1 bis 100.000:1 hinsichtlich der Konzentration des Barcoding-Primers (50) vorhanden ist.

11. Kit zur Genotypisierung eines Lokus (9; 9A, 9B) in einer Zielnukleinsäuresequenz (1A, 1B), wobei der Lokus (9; 9A, 9B) N≥2 Sequenzvarianten aufweist, die N Genotypen entsprechen, wobei das Kit Folgendes umfasst:
N Sätze von Selektionsprimern (10A, 10B, 20; 10, 20A, 20B; 10A, 10B, 20A, 20B), wobei jeder Satz von Selektionsprimern (10A, 10B, 20; 10, 20A, 20B; 10A, 10B, 20A, 20B) der N Sätze von Selektionsprimern (10A, 10B, 20; 10, 20A, 20B; 10A, 10B, 20A, 20B) Folgendes umfasst:
ia) einen jeweiligen Vorwärtsselektionsprimer (10A, 10B), der von einem 5'-Ende (11) zu einem 3'-Ende (12) eine erste nicht-genotypspezifische Amplifikationssequenz (13) und eine jeweilige erste Lokuserkennungssequenz (14A, 14B) umfasst, die komplementär zu einer jeweiligen Sequenzvariante des Lokus (9) sind; und
ib) einen üblichen Rückwärtsselektionsprimer (20), der von einem 5'-Ende (21) zu einem 3'-Ende (22) eine zweite nicht-genotypspezifische Amplifikationssequenz (23) und eine Erkennungssequenz (24) umfasst, die komplementär zu einem zweiten Segment (3) der Zielnukleinsäuresequenz (1) sind;
iia) einen üblichen Vorwärtsselektionsprimer (10), der von einem 5'-Ende (11) zu einem 3'-Ende (12) eine erste nicht-genotypspezifische Amplifikationssequenz (13) und eine Erkennungssequenz (14) umfasst, die komplementär zu einem ersten Segment (2) der Zielnukleinsäuresequenz (1) sind; und
iib) einen jeweiligen Rückwärtsselektionsprimer (20A, 20B), der von einem 5'-Ende (21) zu einem 3'-Ende (22) eine zweite nicht-genotypspezifische Amplifikationssequenz (23) und eine jeweilige zweite Lokuserkennungssequenz (24A, 24B) umfasst, die komplementär zu einer jeweiligen Sequenzvariante des Lokus (9) sind; oder
iiia) einen jeweiligen Vorwärtsselektionsprimer (10A, 10B), der von einem 5'-Ende (11) zu einem 3'-Ende (12) eine erste nicht-genotypspezifische Amplifikationssequenz (13) und eine jeweilige erste Lokuserkennungssequenz (14A, 14B) umfasst, die komplementär zu einer jeweiligen Sequenzvariante eines ersten Lokus (9A) sind; und
iiib) einen jeweiligen Rückwärtsselektionsprimer (20A, 20B), der von einem 5'-Ende (21) zu einem 3'-Ende (22) eine zweite nicht-genotypspezifische Amplifikationssequenz (23) und eine jeweilige zweite Lokuserkennungssequenz (24A, 24B) umfasst, die komplementär zu einer jeweiligen Sequenzvariante eines zweiten Lokus (9B) sind;
einen ersten Satz von Amplifikationsprimern (30, 40), der Folgendes umfasst:
einen Vorwärtsamplifikationsprimer (30), welcher der ersten nicht-genotypspezifischen Amplifikationssequenz (13) entspricht oder komplementär zu dieser ist; und
einen Rückwärtsamplifikationsprimer (40), welcher der zweiten nicht-genotypspezifischen Amplifikationssequenz (23) entspricht oder komplementär zu dieser ist;
i) wobei jeder jeweilige Vorwärtsselektionsprimer (10A, 10B) und der übliche Rückwärtsselektionsprimer (20);
ii) der übliche Vorwärtsselektionsprimer (10) und jeder jeweilige Rückwärtsselektionsprimer (20A, 20B); oder
iii) jeder jeweilige Vorwärtsselektionsprimer (10A, 10B) und jeder jeweilige Rückwärtsselektionsprimer (20A, 20B) in einer Konzentration von weniger als 100 nM vorhanden sind;
wobei der erste Satz von Amplifikationsprimern (30, 40) in einer Konzentration von mindestens 100:1 hinsichtlich einer Konzentration der N Sätze von Selektionsprimern (10A, 10B, 20; 10, 20A, 20B; 10A, 10B, 20A, 20B) vorhanden ist;
N Barcoding-Primer (50A, 50B), wobei jeder Barcoding-Primer (50A, 50B) der N Barcoding-Primer (50A, 50B) von einem 5'-Ende (51) zu einem 3'-Ende (52) Folgendes umfasst:
i) die erste nicht-genotypspezifische Amplifikationssequenz (13) oder eine dritte nicht-genotypspezifische Amplifikationssequenz (53), eine jeweilige Barcode-Sequenz (55A, 55B) und die jeweilige erste Lokuserkennungssequenz (14A, 14B) oder eine jeweilige dritte Lokuserkennungssequenz (54A, 54B), die komplementär zu der jeweiligen Sequenzvariante des Lokus (9) ist/sind; oder
ii) die zweite nicht-genotypspezifische Amplifikationssequenz (23) oder eine dritte nicht-genotypspezifische Amplifikationssequenz (53), eine jeweilige Barcode-Sequenz (55A, 55B) und die jeweilige zweite Lokuserkennungssequenz (24A, 24B) oder eine jeweilige vierte Lokuserkennungssequenz (56A, 56B), die komplementär zu der jeweiligen Sequenzvariante des Lokus (9) ist/sind;
wobei ein zweiter Satz von Amplifikationsprimern (30, 60; 40, 70) Folgendes umfasst:
ia) einen Vorwärtsamplifikationsprimer (30, 60), welcher der ersten nicht-genotypspezifischen Amplifikationssequenz (13) oder der dritten nicht-genotypspezifischen Amplifikationssequenz (53) entspricht oder komplementär zu dieser ist; und
ib) einen Rückwärtsamplifikationsprimer (40), welcher der zweiten nicht-genotypspezifischen Amplifikationssequenz (23) entspricht oder komplementär zu dieser ist; oder
iia) einen Vorwärtsamplifikationsprimer (30), welcher der ersten nicht-genotypspezifischen Amplifikationssequenz (13) entspricht oder komplementär zu dieser ist; und
iib) einen Rückwärtsamplifikationsprimer (40, 70), welcher der zweiten nicht-genotypspezifischen Amplifikationssequenz (23) oder der dritten nicht-genotypspezifischen Amplifikationssequenz (53) entspricht oder komplementär zu dieser ist;
wobei jeder Barcoding-Primer (50A, 50B) in einer Konzentration von weniger als 100 nM vorhanden ist;
wobei der zweite Satz von Amplifikationsprimern (30, 60; 40, 70) in einer Konzentration von mindestens 100:1 hinsichtlich einer Konzentration der *N* Barcoding-Primer (50A, 50B) vorhanden ist; und
*N* markierte Detektionssonden (90A, 90B), die eine jeweilige Markierung (92A, 92B) und eine jeweilige Sequenz (91A, 91B) umfassen, die komplementär zu einer Barcode-Sequenz (55A, 55B) der *N* Barcode-Sequenzen (55A, 55B) ist; oder
eine Detektionsanordnung von *N* genotypspezifischen Sequenzen, wobei jede genotypspezifische Sequenz der *N* genotypspezifischen Sequenzen komplementär zu einer jeweiligen Barcode-Sequenz (55A, 55B) der *N* Barcode-Sequenzen (55A, 55B) ist.

12. Kit nach Anspruch 11, wobei
die *N* markierten Detektionssonden ein jeweiliges markiertes Kügelchen und die jeweilige Sequenz umfassen, die komplementär zu einer Barcode-Sequenz (55A, 55B) der *N* Barcode-Sequenzen (55A, 55B) ist, wobei das Kit ferner Folgendes umfasst:
eine markierte übliche Sonde komplementär zu der ersten nicht-genotypspezifischen Amplifikationssequenz (13) oder der dritten nicht-genotypspezifischen Amplifikationssequenz (53) ist.

13. Kit nach Anspruch 11 oder 12, wobei
i) jeder jeweilige Vorwärtsselektionsprimer (10A, 10B) und der übliche Rückwärtsselektionsprimer (20);
ii) der übliche Vorwärtsselektionsprimer (10) und jeder jeweilige Rückwärtsselektionsprimer (20A, 20B); oder
iii) jeder jeweilige Vorwärtsselektionsprimer (10A, 10B) und jeder jeweilige Rückwärtsselektionsprimer (20A, 20B) in einer Konzentration von 0,01 bis 10 nM vorhanden sind; und
jeder Barcoding-Primer (50A, 50B) in einer Konzentration von 0,01 bis 10 nM vorhanden ist.

14. Kit nach einem der Ansprüche 11 bis 13, wobei der erste Satz von Amplifikationsprimern (30, 40) in einer Konzentration von 100:1 bis 100.000:1 hinsichtlich der Konzentration der *N* Sätze von Selektionsprimern (10A, 10B, 20; 10, 20A, 20B;10A, 10B, 20A, 20B) vorhanden ist; und
der zweite Satz von Amplifikationsprimern (30, 60; 40, 70) in einer Konzentration von 100:1 bis 100.000:1 hinsichtlich der Konzentration der *N* Barcoding-Primer (50A, 50B) vorhanden ist.

15. Kit nach einem der Ansprüche 7 bis 14, wobei ein 5'-Ende des Vorwärtsamplifikationsprimers (30, 60) des zweiten Satzes von Amplifikationsprimern (30, 60; 40, 70) eine Phosphatgruppe umfasst.

## Revendications

1. Procédé d'amplification d'une séquence d'acide nucléique cible (1), ledit procédé comprenant :
a) l'amplification sélective (S2) de ladite séquence d'acide nucléique cible (1) à l'aide d'un ensemble d'amorces de sélection (10, 20) et d'un premier ensemble d'amorces d'amplification (30, 40) dans une réaction enzymatique dépendante des amorces pour produire un amplicon (4), dans lequel
ledit ensemble d'amorces de sélection (10, 20) comprend :
une amorce de sélection directe (10) comprenant, d'une extrémité 5' (11) à une extrémité 3' (12), une première séquence d'amplification non spécifique au génotype (13) et une première séquence de reconnaissance (14) complémentaire à un premier segment (2) de ladite séquence d'acide nucléique cible (1) ; et
une amorce de sélection inverse (20) comprenant, d'une extrémité 5' (21) à une extrémité 3' (22), une deuxième séquence d'amplification non spécifique au génotype (23) et une deuxième séquence de reconnaissance (24) complémentaire à un deuxième segment (3) de ladite séquence d'acide nucléique cible (1) ;
ledit premier ensemble d'amorces d'amplification (30, 40) comprend :
une amorce d'amplification directe (30) correspondant ou complémentaire à ladite première séquence d'amplification non spécifique au génotype (13) ; et
une amorce d'amplification inverse (40) correspondant ou complémentaire à ladite deuxième séquence d'amplification non spécifique au génotype (23) ;
ledit premier ensemble d'amorces d'amplification (30, 40) est présent à une concentration d'au moins 100 : 1 par rapport à la concentration dudit ensemble d'amorces de sélection (10, 20) ; et
b) l'amplification et le codage à barres (S3) dudit amplicon (4) à l'aide d'une amorce de code-barres (50) et d'un deuxième ensemble d'amorces d'amplification (30, 60 ; 40, 70) dans une réaction enzymatique dépendante des amorces pour obtenir un amplicon de code-barres (6), dans lequel
ladite amorce de code-barres (50) comprend, d'une extrémité 5' (51) à une extrémité 3' (52),
i) ladite première séquence d'amplification non spécifique au génotype (13) ou une troisième séquence d'amplification non spécifique au génotype (53), une séquence de code-barres (55) et ladite première séquence de reconnaissance (14) ou une troisième séquence de reconnaissance (54) complémentaire à un premier segment (5) dudit amplicon (4) ; ou
ii) ladite deuxième séquence d'amplification non spécifique au génotype (23) ou une troisième séquence d'amplification non spécifique au génotype (53), une séquence de code-barres (55) et ladite deuxième séquence de reconnaissance (24) ou une quatrième séquence de reconnaissance (56) complémentaire à un deuxième segment (7) dudit amplicon (4) ; ou
iii) ladite amorce de code-barres (50) comprend une amorce de code-barres directe (50') et une amorce de code-barres inverse (50"), ladite amorce de code-barres directe (50') comprenant, de l'extrémité 5' (51') à l'extrémité 3' (52'), ladite première séquence d'amplification non spécifique au génotype (13) ou une troisième séquence d'amplification non spécifique au génotype (53'), une première séquence de code-barres (55') et ladite première séquence de reconnaissance (14) ou une troisième séquence de reconnaissance (54) complémentaire à un premier segment (5) dudit amplicon (4), et ladite amorce de code-barres inverse (50") comprenant, de l'extrémité 5' (51") à l'extrémité 3' (52"), ladite deuxième séquence d'amplification non spécifique au génotype (23) ou une troisième séquence d'amplification non spécifique au génotype (53"), une deuxième séquence de code-barres (55") et ladite deuxième séquence de reconnaissance (24) ou une quatrième séquence de reconnaissance (56) complémentaire à un deuxième segment (7) dudit amplicon (4) ;
ledit deuxième ensemble d'amorces d'amplification (30, 60 ; 40, 70) comprend :
ia) une amorce d'amplification directe (30, 60) correspondant ou complémentaire à ladite première séquence d'amplification non spécifique au génotype (13) ou à ladite troisième séquence d'amplification non spécifique au génotype (53) ; et
ib) une amorce d'amplification inverse (40) correspondant ou complémentaire à ladite deuxième séquence d'amplification non spécifique au génotype (23) ; ou
iia) une amorce d'amplification directe (30) correspondant ou complémentaire à ladite première séquence d'amplification non spécifique au génotype (13) ; et
iib) une amorce d'amplification inverse (40, 70) correspondant ou complémentaire à ladite deuxième séquence d'amplification non spécifique au génotype (23) ou à ladite troisième séquence d'amplification non spécifique au génotype (53) ; ou
iiia) une amorce d'amplification directe (30, 60) correspondant ou complémentaire à ladite première séquence d'amplification non spécifique au génotype (13) ou à ladite troisième séquence d'amplification non spécifique au génotype (53') ; et
iiib) une amorce d'amplification inverse (40, 70) correspondant ou complémentaire à ladite deuxième séquence d'amplification non spécifique au génotype (23) ou à ladite troisième séquence d'amplification non spécifique au génotype (53") ;
ledit deuxième ensemble d'amorces d'amplification (30, 60 ; 40, 70) est présent dans une concentration d'au moins 100 : 1 par rapport à la concentration de ladite amorce de code-barres (50).

2. Procédé de génotypage d'un locus (9 ; 9A, 9B) dans une séquence d'acide nucléique cible (1A, 1B), ledit locus (9 ; 9A, 9B) présentant N ≥ 2 variantes de séquence correspondant à N génotypes, ledit procédé comprenant :
a) l'amplification sélective (S11) de ladite séquence d'acide nucléique cible (1A, 1B) à l'aide de N ensembles d'amorces de sélection (10A, 10B, 20 ; 10, 20A, 20B ; 10A, 10B, 20A, 20B) et d'un premier jeu d'amorces d'amplification (30, 40) dans une réaction enzymatique dépendante des amorces pour produire des amplicons (4A, 4B), dans lequel
chaque ensemble d'amorces de sélection (10A, 10B, 20 ; 10, 20A, 20B ; 10A, 10B, 20A, 20B) des N ensembles d'amorces de sélection (10A, 10B, 20 ; 10, 20A, 20B) comprend :
ia) une amorce de sélection directe respective (10A, 10B) comprenant, d'une extrémité 5' (11) à une extrémité 3' (12), une première séquence d'amplification non spécifique au génotype (13) et une première séquence de reconnaissance de locus respective (14A, 14B) complémentaire à une variante de séquence respective dudit locus (9) ; et
ib) une amorce de sélection inverse commune (20) comprenant, d'une extrémité 5' (21) à une extrémité 3' (22), une deuxième séquence d'amplification non spécifique au génotype (23) et une séquence de reconnaissance (24) complémentaire à un deuxième segment (3) de ladite séquence d'acide nucléique cible (1A, 1B) ;
iia) une amorce de sélection directe commune (10) comprenant, d'une extrémité 5' (11) à une extrémité 3' (12), une première séquence d'amplification non spécifique au génotype (13) et une séquence de reconnaissance (14) complémentaire à un premier segment (2) de ladite séquence d'acide nucléique cible (1A, 1B) ; et
iib) une amorce de sélection inverse respective (20A, 20B) comprenant, d'une extrémité 5' (21) à une extrémité 3' (22), une deuxième séquence d'amplification non spécifique au génotype (23) et une deuxième séquence de reconnaissance de locus respective (24A, 24B) complémentaire à une variante de séquence respective dudit locus (9) ; ou
iiia) une amorce de sélection directe respective (10A, 10B) comprenant, d'une extrémité 5' (11) à une extrémité 3' (12), une première séquence d'amplification non spécifique au génotype (13) et une première séquence de reconnaissance de locus respective (14A, 14B) complémentaire à une variante de séquence respective d'un premier locus (9A) ; et
iiib) une amorce de sélection inverse respective (20A, 20B) comprenant, d'une extrémité 5' (21) à une extrémité 3' (22), une deuxième séquence d'amplification non spécifique au génotype (23) et une deuxième séquence de reconnaissance de locus respective (24A, 24B) complémentaire à une variante de séquence respective d'un deuxième locus (9B) ;
ledit premier ensemble d'amorces d'amplification (30, 40) comprend :
une amorce d'amplification directe (30) correspondant ou complémentaire à ladite première séquence d'amplification non spécifique au génotype (13) ; et
une amorce d'amplification inverse (40) correspondant ou complémentaire à ladite deuxième séquence d'amplification non spécifique au génotype (23) ;
ledit premier ensemble d'amorces d'amplification (30, 40) est présent dans une concentration d'au moins 100 : 1 par rapport à une concentration desdits N ensembles d'amorces de sélection (10A, 10B, 20 ; 10, 20A, 20B; 10A, 10B, 20A, 20B) ;
b) l'amplification et le codage à barres (S12) desdits amplicons (4A, 4B) à l'aide de N amorces de code-barres (50A, 50B) et d'un deuxième ensemble d'amorces d'amplification (30, 60 ; 40, 70) dans une réaction enzymatique dépendante des amorces pour produire des amplicons codés à barres (6A, 6B), dans lequel chaque amorce de code-barres (50A, 50B) desdites N amorces de code-barres (50A, 50B) comprend, d'une extrémité 5' (51) à une extrémité 3' (52),
i) ladite première séquence d'amplification non spécifique au génotype (13) ou une troisième séquence d'amplification non spécifique au génotype (53), une séquence de code-barres respective (55A, 55B) et ladite première séquence de reconnaissance de locus respective (14A, 14B) ou une troisième séquence de reconnaissance de locus respective (54A, 54B) complémentaire à ladite variante de séquence respective dudit locus (9 ; 9A) ; ou
ii) ladite deuxième séquence d'amplification non spécifique au génotype (23) ou une troisième séquence d'amplification non spécifique au génotype (53), une séquence de code-barres respective (55A, 55B) et ladite deuxième séquence de reconnaissance de locus respective (24A, 24B) ou une quatrième séquence de reconnaissance de locus respective (56A, 56B) complémentaire à ladite variante de séquence respective dudit locus (9 ; 9B) ;
ledit deuxième ensemble d'amorces d'amplification (30, 60 ; 40, 70) comprend :
ia) une amorce d'amplification directe (30, 60) correspondant ou complémentaire à ladite première séquence d'amplification non spécifique au génotype (13) ou à ladite troisième séquence d'amplification non spécifique au génotype (53) ; et
ib) une amorce d'amplification inverse (40) correspondant ou complémentaire à ladite deuxième séquence d'amplification non spécifique au génotype (23) ; ou
iia) une amorce d'amplification directe (30) correspondant ou complémentaire à ladite première séquence d'amplification non spécifique au génotype (13) ; et
iib) une amorce d'amplification inverse (40, 70) correspondant ou complémentaire à ladite deuxième séquence d'amplification non spécifique au génotype (23) ou à ladite troisième séquence d'amplification non spécifique au génotype (53) ;
ledit deuxième ensemble d'amorces d'amplification (30, 60 ; 40, 70) est présent à une concentration d'au moins 100 : 1 par rapport à la concentration desdites N amorces de code-barres (50A, 50B) ; et
c) le génotypage (S13) desdites N variantes de séquence dudit locus (9 ; 9A, 9B) en :
c1a) contactant, pendant ou après l'amplification et le codage à barres de l'étape b), chaque amplicon codé à barres (6A, 6B) avec N sondes de détection marquées (90A, 90B) comprenant une étiquette respective (92A, 92B) et une séquence respective (91A, 91B) complémentaire à une séquence de code-barres (55A, 55B) desdites N séquences de code-barres (55A, 55B) ; et
c1b) reliant une quantité d'étiquette détectée (92A, 92B) à un génotype spécifique pour ledit locus (9 ; 9A, 9B) ; ou
c2a) contactant, après amplification et codage à barres à l'étape b), chaque amplicon codé à barres (6A, 6B) avec une matrice de détection de N séquences spécifiques de génotype, chaque séquence spécifique de génotype desdites N séquences spécifiques de génotype étant complémentaire à une séquence de code-barres respective (55A, 55B) desdites N séquences de code-barres (55A, 55B) ; et
c2b) reliant l'hybridation détectée de chaque amplicon à code-barres (6A, 6B) à ladite matrice de détection à un génotype spécifique pour ledit locus (9; 9A, 9B).

3. Procédé selon la revendication 2, dans lequel le contact de chaque amplicon à code-barres à l'étape c1a) comprend le contact, pendant ou après l'amplification et le codage à barres à l'étape b), de chaque amplicon à code-barres (6A, 6B) avec une sonde commune marquée complémentaire à ladite première séquence d'amplification non spécifique au génotype (13) ou à ladite troisième séquence d'amplification non spécifique au génotype (53) et N sondes de détection marquées comprenant une bille marquée respective et ladite séquence respective complémentaire à une séquence de code-barres (55A, 55B) desdites N séquences de code-barres (55A, 55B) ; et
la mise en relation de ladite quantité d'étiquette détectée à l'étape c1b) comprend la mise en relation d'une quantité de bille marquée détectée avec ledit génotype spécifique pour ledit locus (9 ; 9A, 9B).

4. Procédé selon l'une quelconque des revendications 1 à 3, comprenant en outre une préamplification (S1, S10) de ladite séquence d'acide nucléique cible (1 ; 1A, 1B) dans une réaction enzymatique dépendante d'une amorce.

5. Procédé selon la revendication 4, dans lequel la préamplification (S1, S10) de ladite séquence d'acide nucléique cible (1 ; 1A, 1B) comprend :
l'amplification par déplacement multiple de ladite séquence d'acide nucléique cible (1 ; 1A, 1B) dans une réaction enzymatique dépendante d'une amorce ; ou
l'amplification indépendante de la séquence de ladite séquence d'acide nucléique cible (1 ; 1A, 1B) dans une réaction enzymatique dépendante d'une amorce.

6. Procédé selon l'une quelconque des revendications 1 à 5, dans lequel l'extrémité 5' de ladite amorce d'amplification directe (60) dudit deuxième ensemble d'amorces d'amplification (30, 60 ; 40, 70) comprend un groupe phosphate, ledit procédé comprend en outre :
la génération (S20) d'amplicons à code-barres simple brin (6A, 6B) en traitant lesdits amplicons à code-barres (6A, 6B) après l'étape b) avec une exonucléase.

7. Kit pour l'amplification d'une séquence d'acide nucléique cible (1), ledit kit comprenant :
un ensemble d'amorces de sélection (10, 20) comprenant :
une amorce de sélection directe (10) comprenant, d'une extrémité 5' (11) à une extrémité 3' (12), une première séquence d'amplification non spécifique au génotype (13) et une première séquence de reconnaissance (14) complémentaire à un premier segment (2) de ladite séquence d'acide nucléique cible (1) ; et
une amorce de sélection inverse (20) comprenant, d'une extrémité 5' (21) à une extrémité 3' (22), une deuxième séquence d'amplification non spécifique au génotype (23) et une deuxième séquence de reconnaissance (24) complémentaire à un deuxième segment (3) de ladite séquence d'acide nucléique cible (1) ;
un premier ensemble d'amorces d'amplification (30, 40) comprenant :
une amorce d'amplification directe (30) correspondant ou complémentaire à ladite première séquence d'amplification non spécifique au génotype (13) ; et
une amorce d'amplification inverse (40) correspondant ou complémentaire à ladite deuxième séquence d'amplification non spécifique au génotype (23) ;
l'amorce de sélection directe (10) et l'amorce de sélection inverse (20) sont présentes dans une concentration inférieure à 100 nM ;
ledit premier ensemble d'amorces d'amplification (30, 40) est présent à une concentration d'au moins 100 : 1 par rapport à la concentration dudit ensemble d'amorces de sélection (10, 20) ; et
une amorce de code-barres (50) comprenant, d'une extrémité 5' (51) à une extrémité 3' (52),
i) ladite première séquence d'amplification non spécifique au génotype (13) ou une troisième séquence d'amplification non spécifique au génotype (53), une séquence de code-barres (55) et ladite première séquence de reconnaissance (14) ou une troisième séquence de reconnaissance (54) complémentaire à un premier segment (5) dudit amplicon (4) ; ou
ii) ladite deuxième séquence d'amplification non spécifique au génotype (23) ou une troisième séquence d'amplification non spécifique au génotype (53), une séquence de code-barres (55) et ladite deuxième séquence de reconnaissance (24) ou une quatrième séquence de reconnaissance (56) complémentaire à un deuxième segment (7) dudit amplicon (4) ; ou
iii) ladite amorce de code-barres (50) comprend une amorce de code-barres directe (50') et une amorce de code-barres inverse (50"), ladite amorce de code-barres directe (50') comprenant, de l'extrémité 5' (51') à l'extrémité 3' (52'), ladite première séquence d'amplification non spécifique au génotype (13) ou une troisième séquence d'amplification non spécifique au génotype (53'), une première séquence de code-barres (55') et ladite première séquence de reconnaissance (14) ou une troisième séquence de reconnaissance (54) complémentaire à un premier segment (5) dudit amplicon (4), et ladite amorce de code-barres inverse (50") comprenant, de l'extrémité 5' (51") à l'extrémité 3' (52"), ladite deuxième séquence d'amplification non spécifique au génotype (23) ou une troisième séquence d'amplification non spécifique au génotype (53"), une deuxième séquence de code-barres (55") et ladite deuxième séquence de reconnaissance (24) ou une quatrième séquence de reconnaissance (56) complémentaire à un deuxième segment (7) dudit amplicon (4) ;
un deuxième ensemble d'amorces d'amplification (30, 60 ; 40, 70) comprenant :
ia) une amorce d'amplification directe (30, 60) correspondant ou complémentaire à ladite première séquence d'amplification non spécifique au génotype (13) ou à ladite troisième séquence d'amplification non spécifique au génotype (53) ; et
ib) une amorce d'amplification inverse (40) correspondant ou complémentaire à ladite deuxième séquence d'amplification non spécifique au génotype (23) ; ou
iia) une amorce d'amplification directe (30) correspondant ou complémentaire à ladite première séquence d'amplification non spécifique au génotype (13) ; et
iib) une amorce d'amplification inverse (40, 70) correspondant ou complémentaire à ladite deuxième séquence d'amplification non spécifique au génotype (23) ou à ladite troisième séquence d'amplification non spécifique au génotype (53) ; ou
iiia) une amorce d'amplification directe (30, 60) correspondant ou complémentaire à ladite première séquence d'amplification non spécifique au génotype (13) ou à ladite troisième séquence d'amplification non spécifique au génotype (53') ; et
iiib) une amorce d'amplification inverse (40, 70) correspondant ou complémentaire à ladite deuxième séquence d'amplification non spécifique au génotype (23) ou à ladite troisième séquence d'amplification non spécifique au génotype (53") ;
ladite amorce de code-barres (50) est présente à une concentration inférieure à 100 nM ; et
ledit deuxième ensemble d'amorces d'amplification (30, 60 ; 40, 70) est présent dans une concentration d'au moins 100 : 1 par rapport à la concentration de ladite amorce de code-barres (50).

8. Kit selon la revendication 7, dans lequel
ladite amorce de code-barres (50) comprend, de ladite extrémité 5' (51) à ladite extrémité 3' (52), ladite première séquence d'amplification non spécifique au génotype (13) ou ladite troisième séquence d'amplification non spécifique au génotype (53), de préférence ladite troisième séquence d'amplification non spécifique au génotype (53), ladite séquence de code-barres (55) et ladite première séquence de reconnaissance (14) ou ladite troisième séquence de reconnaissance (54) ; et
ledit deuxième ensemble d'amorces d'amplification (30, 60 ; 40,
70) comprend :
ladite amorce d'amplification directe (30, 60) correspondant ou complémentaire à ladite première séquence d'amplification non spécifique au génotype (13) ou à ladite troisième séquence d'amplification non spécifique au génotype (53), de préférence à ladite troisième séquence d'amplification non spécifique au génotype (53) ; et
ladite amorce d'amplification inverse (40) correspondant ou complémentaire à ladite deuxième séquence d'amplification non spécifique au génotype (23).

9. Kit selon la revendication 7 ou 8, dans lequel l'amorce de sélection directe (10) et l'amorce de sélection inverse (20) sont présentes dans une concentration de 0.01 à 10 nM ;
ladite amorce de code-barres (50) est présente à une concentration 0.01 à 10 nM ; et

10. Procédé selon l'une quelconque des revendications 7 à 9, dans lequel
ledit premier ensemble d'amorces d'amplification (30, 40) est présent dans une concentration de 100 : 1 à 100000 : 1 par rapport à ladite concentration dudit ensemble d'amorces de sélection (10, 20) ; et
ledit deuxième ensemble d'amorces d'amplification (30, 60 ; 40, 70) est présent dans une concentration de 100 : 1 à 100000 : 1 par rapport à ladite concentration de ladite amorce de code-barres (50).

11. Kit de génotypage d'un locus (9 ; 9A, 9B) dans une séquence d'acide nucléique cible (1A, 1B), ledit locus (9 ; 9A, 9B) présentant N ≥ 2 variantes de séquence correspondant à N génotypes, ledit kit comprenant :
N ensembles d'amorces de sélection (10A, 10B, 20 ; 10, 20A, 20B ; 10A, 10B, 20A, 20B), chaque ensemble d'amorces de sélection (10A, 10B, 20 ; 10, 20A, 20B ; 10A, 10B, 20A, 20B) des N ensembles d'amorces de sélection (10A, 10B, 20 ; 10, 20A, 20B ; 10A, 10B, 20A, 20B) comprenant :
ia) une amorce de sélection directe respective (10A, 10B) comprenant, d'une extrémité 5' (11) à une extrémité 3' (12), une première séquence d'amplification non spécifique au génotype (13) et une première séquence de reconnaissance de locus respective (14A, 14B) complémentaire à une variante de séquence respective dudit locus (9) ; et
ib) une amorce de sélection inverse commune (20) comprenant, d'une extrémité 5' (21) à une extrémité 3' (22), une deuxième séquence d'amplification non spécifique au génotype (23) et une séquence de reconnaissance (24) complémentaire à un deuxième segment (3) de ladite séquence d'acide nucléique cible (1) ;
iia) une amorce de sélection directe commune (10) comprenant, d'une extrémité 5' (11) à une extrémité 3' (12), une première séquence d'amplification non spécifique au génotype (13) et une séquence de reconnaissance (14) complémentaire à un premier segment (2) de ladite séquence d'acide nucléique cible (1) ; et
iib) une amorce de sélection inverse respective (20A, 20B) comprenant, d'une extrémité 5' (21) à une extrémité 3' (22), une deuxième séquence d'amplification non spécifique au génotype (23) et une deuxième séquence de reconnaissance de locus respective (24A, 24B) complémentaire à une variante de séquence respective dudit locus (9) ; ou
iiia) une amorce de sélection directe respective (10A, 10B) comprenant, d'une extrémité 5' (11) à une extrémité 3' (12), une première séquence d'amplification non spécifique au génotype (13) et une première séquence de reconnaissance de locus respective (14A, 14B) complémentaire à une variante de séquence respective d'un premier locus (9A) ; et
iiib) une amorce de sélection inverse respective (20A, 20B) comprenant, d'une extrémité 5' (21) à une extrémité 3' (22), une deuxième séquence d'amplification non spécifique au génotype (23) et une deuxième séquence de reconnaissance de locus respective (24A, 24B) complémentaire à une variante de séquence respective d'un deuxième locus (9B) ;
un premier ensemble d'amorces d'amplification (30, 40) comprenant :
une amorce d'amplification directe (30) correspondant ou complémentaire à ladite première séquence d'amplification non spécifique au génotype (13) ; et
une amorce d'amplification inverse (40) correspondant ou complémentaire à ladite deuxième séquence d'amplification non spécifique au génotype (23) ;
i) chaque amorce de sélection directe respective (10A, 10B) et ladite amorce de sélection inverse commune (20) ;
ii) ladite amorce de sélection directe commune (10) et chaque amorce de sélection inverse respective (20A, 20B) ; ou
iii) chaque amorce de sélection directe respective (10A, 10B) et chaque amorce de sélection inverse respective (20A, 20B) est présente à une concentration inférieure à 100 nM ;
ledit premier ensemble d'amorces d'amplification (30, 40) est présent dans une concentration d'au moins 100 : 1 par rapport à une concentration desdits *N* ensembles d'amorces de sélection (10A, 10B, 20 ; 10, 20A, 20B; 10A, 10B, 20A, 20B) ;
N amorces de codage à barres (50A, 50B), chaque amorce de codage à barres (50A, 50B) desdites *N* amorces de codage à barres (50A, 50B) comprenant, d'une extrémité 5' (51) à une extrémité 3' (52),
i) ladite première séquence d'amplification non spécifique au génotype (13) ou une troisième séquence d'amplification non spécifique au génotype (53), une séquence de code-barres respective (55A, 55B) et ladite première séquence de reconnaissance de locus respective (14A, 14B) ou une troisième séquence de reconnaissance de locus respective (54A, 54B) complémentaire à ladite variante de séquence respective dudit locus (9) ; ou
ii) ladite deuxième séquence d'amplification non spécifique au génotype (23) ou une troisième séquence d'amplification non spécifique au génotype (53), une séquence de code-barres respective (55A, 55B) et ladite deuxième séquence de reconnaissance de locus respective (24A, 24B) ou une quatrième séquence de reconnaissance de locus respective (56A, 56B) complémentaire à ladite variante de séquence respective dudit locus (9) ;
un deuxième ensemble d'amorces d'amplification (30, 60 ; 40, 70) comprend :
ia) une amorce d'amplification directe (30, 60) correspondant ou complémentaire à ladite première séquence d'amplification non spécifique au génotype (13) ou à ladite troisième séquence d'amplification non spécifique au génotype (53) ; et
ib) une amorce d'amplification inverse (40) correspondant ou complémentaire à ladite deuxième séquence d'amplification non spécifique au génotype (23) ; ou
iia) une amorce d'amplification directe (30) correspondant ou complémentaire à ladite première séquence d'amplification non spécifique au génotype (13) ; et
iib) une amorce d'amplification inverse (40, 70) correspondant ou complémentaire à ladite deuxième séquence d'amplification non spécifique au génotype (23) ou à ladite troisième séquence d'amplification non spécifique au génotype (53) ;
chaque amorce de code-barres (50A, 50B) est présente à une concentration inférieure à 100 nM ;
ledit deuxième ensemble d'amorces d'amplification (30, 60 ; 40, 70) est présent à une concentration d'au moins 100 : 1 par rapport à la concentration desdites N amorces de code-barres (50A, 50B) ; et
N sondes de détection marquées (90A, 90B) comprenant une étiquette respective (92A, 92B) et une séquence respective (91A, 91B) complémentaire à une séquence de code-barres (55A, 55B) desdites *N* séquences de code-barres (55A, 55B) ; ou
un réseau de détection de *N* séquences spécifiques de génotype, chaque séquence spécifique de génotype desdites N séquences spécifiques de génotype est complémentaire à une séquence de code-barres respective (55A, 55B) desdites N séquences de code-barres (55A, 55B).

12. Kit selon la revendication 11, dans lequel
lesdites *N* sondes de détection marquées comprennent une bille marquée respective et ladite séquence respective complémentaire à une séquence de code-barres (55A, 55B) desdites *N* séquences de code-barres (55A, 55B), ledit kit comprenant en outre :
une sonde commune marquée complémentaire à ladite première séquence d'amplification non spécifique au génotype (13) ou à ladite troisième séquence d'amplification non spécifique au génotype (53).

13. Kit selon la revendication 11 ou 12, dans lequel
i) chaque amorce de sélection directe respective (10A, 10B) et ladite amorce de sélection inverse commune (20) ;
ii) ladite amorce de sélection directe commune (10) et chaque amorce de sélection inverse respective (20A, 20B) ; ou
iii) chaque amorce de sélection directe respective (10A, 10B) et chaque amorce de sélection inverse respective (20A, 20B) est présente à une concentration de 0,01 à 10 nM ; et
chaque amorce de code-barres (50A, 50B) est présente à une concentration 0.01 à 10 nM.

14. Procédé selon l'une quelconque des revendications 11 à 13, dans lequel
ledit premier ensemble d'amorces d'amplification (30, 40) est présent à une concentration de 100 : 1 à 100000 : 1 par rapport à ladite concentration des *N* ensembles d'amorces de sélection (10A, 10B, 20 ; 10, 20A, 20B ; 10A, 10B, 20A, 20B) ; et
ledit deuxième ensemble d'amorces d'amplification (30, 60 ; 40, 70) est présent dans une concentration de 100 : 1 à 100000 : 1 par rapport à ladite concentration desdites *N* amorces de code-barres (50A, 50B).

15. Kit selon l'une quelconque des revendications 7 à 14, dans lequel une extrémité 5' de ladite amorce d'amplification directe (30, 60) dudit deuxième ensemble d'amorces d'amplification (30, 60 ; 40, 70) comprend un groupe phosphate.
